# EUROPÄISCHE PATENTSCHRIFT

(11) **EP 2 037 974 B1**
(45) Veröffentlichungstag und Bekanntmachung des Hinweises auf die Patenterteilung: **25.05.2011**
(21) Anmeldenummer: 07785970.0
(22) Anmeldetag: 10.07.2007
(51) Int. Cl.: A61L 27/22, A61K 47/42, A61L 27/38

(54) **VERWENDUNG VON GELATINE UND EINEM VERNETZUNGSMITTEL ZUR HERSTELLUNG EINER VERNETZENDEN THERAPEUTISCHEN ZUSAMMENSETZUNG**
USE OF GELATIN AND A CROSS-LINKING AGENT FOR PRODUCING A CROSS-LINKING THERAPEUTIC COMPOSITION
UTILISATION DE GÉLATINE ET D'UN AGENT RÉTICULANT POUR PRODUIRE UNE COMPOSITION THÉRAPEUTIQUE À RÉTICULATION

(30) Priorität: 10.07.2006 DE 102006033168
(43) Veröffentlichungstag der Anmeldung: 25.03.2009
(73) Patentinhaber: TETEC Tissue Engineering Technologies AG, 72770 Reutlingen (DE)
(72) Erfinder: GAISSMAIER, Christoph, 72127 Kusterdingen (DE); AHLERS, Michael, 69412 Eberbach (DE)
(74) Vertreter: Patentanwälte Ruff, Wilhelm, Beier, Dauster & Partner
(86) Internationale Anmeldenummer: PCT/EP2007/006104
(87) Internationale Veröffentlichungsnummer: WO 2008/006544

(56) Entgegenhaltungen:
- WO-A-96/40304
- WO-A-2005/111121
- WO-A-2007/057176
- WO-A-2008/006545
- JP-A- 2004 283 371
- US-A- 6 007 613
- CHEN T ET AL: "Enzyme-catalyzed gel formation of gelatin and chitosan: potential for in situ applications" BIOMATERIALS, ELSEVIER SCIENCE PUBLISHERS BV., BARKING, GB, Bd. 24, Nr. 17, 1. August 2003 (2003-08-01), Seiten 2831-2841, XP004424096 ISSN: 0142-9612
- AKIRA ITO, ATSUNORI MASE, YOHEI TAKIZAWA, MASASHIGE SHINKAI, HIROYUKI HONDA, KEN-ICHIRO HATA, MINORU UEDA, TAKESHI KOBAYASHI: "Transglutaminase-mediated gelatin matrices incorporating cell adhesion factors as a biomaterial for tissue engineering" JOURNAL OF BIOSCIENCE AND BIOENGINEERING, Bd. 95, Nr. 2, 2003, Seiten 196-199, XP002494711

## Beschreibung

Die vorliegende Erfindung betrifft eine neuartige Verwendung von Gelatine und Transglutaminase zur Herstellung einer vernetzenden therapeutischen Zusammensetzung zur Behandlung von Bandscheiben- oder Meniskusschäden, die Zusammensetzung ein vernetztes Gelatinegel als Zellmatrix in einem Zielbereich des menschlichen Behandlung von Bandscheiben - oder Meniskusschäden, die ein vernetztes Gelatinegel als Zellmatrix in einem Zielbereich des menschlichen oder tierischen Körpers bildet, wobei der Zielbereich der Nucleus pulposus oder der Annulus fibrosus einer Bandscheibe oder ein Meniskus ist und wobei die Zusammensetzung Bandscheibenzellen, Chondrozyten oder mesenchymale Stammzellen umfasst.

In verschiedenen Bereichen der Medizin kommen körperverträgliche, biologisch abbaubare Matrixmaterialien zum Einsatz. Eine bedeutende Rolle spielen dabei therapeutische Anwendungen, bei denen das biologisch abbaubare Material als Zellmatrix dient, d.h. als eine das Wachstum, die Proliferation und/oder die Differenzierung von Zellen unterstützende Matrix. Hierunter fallen sowohl Anwendungen, bei denen das Matrixmaterial zellfrei und gegebenenfalls in Verbindung mit Wachstumsfaktoren appliziert oder verabreicht wird, um im Ziel bereich des Körpers eine wachstums- bzw. regenerationsfördernde Funktion zu erfüllen, als auch Anwendungen, bei denen das Matrixmaterial bereits *in vitro* mit Zellen besiedelt wird. Bei den letztgenannten Anwendungen kann eine Vorkultivierung der Zellen in bzw. auf dem Matrixmaterial *in vitro* durchgeführt werden, wobei ein so genanntes Gewebeimplantat gebildet wird, welches dann an der zu behandelnden Stelle des Körpers eingesetzt wird. Als Beispiel für die beschriebenen Verfahren ist die Behandlung von Knochendefekten mit Biomaterialien und Wachstumsfaktoren (z.B thrombozytäre Wachstumsfaktoren oder BMPs (Bone Morphogentic Proteins)) oder die Behandlung von Knorpeldefekten mit Hilfe der autologen oder allogenen Knorpelzelltransplantation zu nennen.

Die für die genannten Zwecke am häufigsten verwendeten Matrixmaterialien sind Biopolymere auf Basis von Proteinen oder Polysacchariden, insbesondere Kollagen, Gelatine, Hyaluronsäure, Chitosan oder Alginate. Bevorzugte Anwendungsformen dieser Materialien sind Gele oder Schwammstrukturen, die von ihrer Struktur her eine möglichst gleichmäßige Verteilung der Zellen ermöglichen. Bei löslichen Polymeren, wie z.B. Gelatine, müssen diese in der Regel in vernetzter Form eingesetzt werden, um Matrizes herstellen zu können, die unter physiologischen Bedingungen formstabil sind und eine ausreichend lange Lebensdauer aufweisen. Derartige Formkörper auf Basis von vernetzter Gelatine sind beispielsweise in der deutschen Patentanmeldung DE 10 2004 024 635 A1 beschrieben.

WO96/40304 beschreibt eine injizierbare Polymerzusammensetzung die Zellen enthält. Die Komposition vernetzt in vivo, zur Formung einer Zellmatrix im menschlichen oder tierischen Körper, in einem Zielbereich der Regeneration braucht. Das Polymer der Polymerkomposition kann Gelatine umfassen.

T. Chen et al. offenbart die Bildung eines vernetzten Gelatinegels in einem Zielbereich des Körpers, das als Zellmatrix benutzt werden kann. Diese Matrix formt sich in situ durch die Vernetzung der Gelatine, nach der Addition von Transglutaminase (Biomaterials, Elsevier Science Publishers BV., BARKING, GB, Bd. 24, Nr. 17, 1. August 2003 (2003-08-01), Seiten 2831-2841 ).

JP2004283371 offenbart eine Zusammensetzung die Gelatine, Zellen und Transglutaminase umfasst. Die Komposition formt ein Gelatinegel als Zellmatrix für Regeneration von Gewebe

A. Ito et al. beschreibt vernetzte Gelatine, die als Zellmatrix im menschlichen oder tierischen Körper benutzt werden kann. Die Gelatine wird vernetzt mit Transglutaminase. Lebende Zellen, wie Fibroblasten, und Wachstums- oder Differenzierungsfaktoren können auch in der Gelatinematrix integriert werden. Die Komposition die die Gelmatrix bildet wird (Journal of Bioscience and Bioengineering, Bd. 95, Nr. 2, 2003, Seiten 196-199).

Eine weitere Indikation, bei der eine Behandlung mit gewebespezifischen Zellen in Frage kommt, ist die Schädigung bzw. Degeneration der Bandscheibe, insbesondere des Nucleus pulposus (Gallertkern). Eine Degeneration des Gewebes des Nucleus pulposus, die mit zunehmendem Alter mit erhöhter Wahrscheinlichkeit auftreten kann, führt zu einer höheren Belastung des Annulus fibrosus (Faserring), was letztlich zu dessen Schädigung und somit zu einem Bandscheibenvorfall führen kann.

Die Bandscheibenzellen des Nucleus pulposus verfügen über keine ausreichende Fähigkeit, sich selbst zu regenerieren. Um den oben beschriebenen Folgen rechtzeitig vorzubeugen, wird daher das Verfahren der biologischen Bandscheibenrekonstruktion eingesetzt. Dabei werden Bandscheibenzellen oder mesenchymale Stammzellen *in vitro* kultiviert und anschließend in den Nucleus pulposus des Patienten verabreicht. Eine weitere Möglichkeit besteht darin, geeignete Wachstums- und Differenzierungsfaktoren mit Hilfe eines Biomaterials in die Bandscheibe zu applizieren, die dort eine lokal begrenzte Wirksamkeit entfalten und so zur Regeneration der Bandscheibe beitragen. Allerdings ist es nicht möglich, eine feste, mit Wachstumsfaktoren kombinierte oder mit Zellen besiedelte Matrix in den Nucleus pulposus zu implantieren, da dies unweigerlich mit einer erheblichen Beschädigung des Annulus fibrosus verbunden wäre.

Aus diesem Grund werden die Zellen, die Wachstumsfaktoren oder eine Kombination von beidem in einem flüssigen Medium, z.B. in einer Nährlösung oder dergleichen, in die Bandscheibe injiziert. Ein Problem besteht allerdings darin, dass sich der Injektionsweg nicht ausreichend verschließen lässt und somit die in dem flüssigen Medium suspendierten Zellen und/oder Wachstumsfaktoren durch den Injektionsweg wieder aus der Bandscheibe herausgepresst werden können, sobald Druck auf diese ausgeübt wird.

Wünschenswert wäre daher für diese und ähnliche biologische Regenerationsverfahren eine therapeutische Zusammensetzung, die an einen Zielbereich des Körpers verabreicht werden kann, wobei sichergestellt ist, dass die suspendierten Zellen und/oder die Wachstumsfaktoren im Zielbereich des Körpers verbleiben, ohne dass dem Patienten eine unzumutbar lange Ruhigstellung des behandelten Körperbereichs abverlangt werden muss.

Zur Lösung der genannten Probleme wird erfindungsgemäß die Verwendung von Gelatine und Transglutaminase zur Herstellung einer vernetzenden therapeutischen Zusammensetzung der eingangs genannten Art vorgeschlagen, die im Zielbereich ein vernetztes Gelatinegel als Zellmatrix bildet, wobei
(i) die Gelatine und die Transglutaminase zur Bildung der vernetzenden therapeutischen Zusammensetzung miteinander gemischt werden, welche anschließend in den Zielbereich verabreicht wird; oder
(ii) die Gelatine und die Transglutaminase in voneinander getrennter Form bereitgestellt und gleichzeitig oder aufeinander folgend unter Bildung der vernetzenden therapeutischen Zusammensetzung in den Zielbereich verabreicht werden.

Der Erfindung liegt die Idee zugrunde, als biologisch abbaubares Matrixmaterial Gelatine einzusetzen, die sich durch ihre Wasserlöslichkeit auszeichnet, und die somit in Form einer Lösung verabreicht werden kann, insbesondere durch Injektion. Im Zielbereich erfolgt dann der Übergang der löslichen Gelatine in ein Gelatinegel, welches durch die Einwirkung des Vernetzungsmittels in eine unlösliche Form, d.h. in ein vernetztes Gelatinegel, überführt wird, welches eine mechanisch belastbare Zellmatrix bildet.

Die erfindungsgemäße therapeutische Zusammensetzung benötigt außer der Gelatine und dem Vernetzungsmittel keine weiteren Komponenten, die zur Bildung des vernetzten Gelatinegels beitragen. Dies schließt allerdings die Anwesenheit weiterer Komponenten nicht aus, durch die z.T. weitere vorteilhafte Effekte erzielt werden können.

Bei den Zellen, denen das Gelatinegel als Matrix dient, kann es sich einerseits um lebende Zellen handeln, die dem Patienten gemeinsam mit der therapeutischen Zusammensetzung verabreicht werden. Das Gelatinegel kann aber auch als Matrix für das Einwachsen der bereits im Zielbereich befindlichen körpereigenen Zellen dienen, wobei die therapeutische Zusammensetzung dann bevorzugt Wachstums- und/oder Differenzierungsfaktoren, die die Regeneration dieser Zellen bzw. des durch sie gebildeten Gewebes fördern, umfasst.

Das oben genannte Problem wird durch die erfindungsgemäße therapeutische Zusammensetzung insofern gelöst, dass das vernetzte Gelatinegel eine feste Matrix bereitstellt, die ein Herauspressen der Zellen, der Wachstumsfaktoren oder der Kombination von Zellen mit Wachstumsfaktoren aus dem Zielbereich weitgehend verhindert. Zusätzlich bietet die erfindungsgemäße therapeutische Zusammensetzung durch die Ausbildung eines Gelatinegels den Vorteil, dass sie eine im Wesentlichen gleichmäßige Verteilung von Wirkstoffen und Zellen in einem dreidimensionalen Bereich ermöglicht und stabilisiert, im Gegensatz zu flüssigen Medien, in denen Zellen in Folge der Schwerkraft absinken. Ein weiterer Vorteil der vernetzten und dann unlöslichen Gelatine ist, dass sie die applizierten Wachstumsfaktoren und/oder anderen therapeutischen Wirkstoffe im Zielgewebe bindet bzw. fixiert und so eine lokal begrenzte Wirkungsentfaltung im Sinne einer kontrollierten und kontinuierlichen Wirkstofffreisetzung überhaupt erst ermöglicht. Hierdurch können unerwünschte Nebenwirkungen, die durch ein wieder Austreten von Zellen oder Wirkstoffen aus der Bandscheibe verursacht werden könnten, verhindert werden.

Die zu kultivierenden Zellen sowie gegebenenfalls Wachstumsfaktoren werden der vernetzenden Zusammensetzung zugegeben, bevor eine wesentliche Vernetzung der Gelatine erfolgt. Bevorzugt wird eine die Zellen und die gelöste Gelatine enthaltende Kultur- oder Nährlösung bereitgestellt, die vor oder nach dem Einfüllen in das Kulturgefäß, d.h. den Zielbereich, mit dem Vernetzungsmittel versetzt wird.

Bevor die verschiedenen Varianten der Bereitstellung und Verabreichung der Gelatine und des Vernetzungsmittels im Detail beschrieben werden, soll zunächst auf die besonderen Vorteile eingegangen werden, die sich durch die Auswahl von Gelatine als Matrixmaterial ergeben.

Gelatine ist, im Gegensatz zu Kollagen, in definierter und reproduzierbarer Zusammensetzung sowie in hoher Reinheit erhältlich. Insbesondere enthält sie praktisch keine immunogenen Telopeptide, die Abwehrreaktionen des Körpers auslösen könnten. Insofern weist Gelatine eine hervorragende Gewebe- und Zellverträglichkeit auf, wie sie von anderen resorbierbaren Biomaterialien wie Alginaten oder Chitosan nicht gewährleistet werden kann.

Während unvernetzte Gelatine bei Körpertemperatur (37°C) löslich ist, kann sie, wie bereits erwähnt, durch Vernetzung in eine unter diesen Bedingungen unlösliche gelartige Form, d.h. ein vernetztes Gelatinegel, überführt werden. Ein solches Gelatinegel kann als Zellmatrix für das Wachstum und die Differenzierung von Zellen dienen.

Gleichzeitig ist das vernetzte Gelatinegel vollständig resorbierbar, d.h. es wird nach einer bestimmten Zeit im Körper rückstandslos abgebaut. Dabei handelt es sich um einen hydrolytischen Abbau, der gegebenenfalls durch körpereigene Enzyme unterstützt wird.

Grundsätzlich kann im Rahmen der vorliegenden Erfindung Gelatine verschiedener Herkunft eingesetzt werden, wobei porcine Gelatine bevorzugt ist, insbesondere Schweineschwartengelatine. Diese ist in hoher Qualität erhältlich und ist bereits für verschiedene medizinische Anwendungen zugelassen.

Daneben kann auch die Verwendung anderer Gelatinearten, wie z.B. Fischgelatine, besondere Vorteile bieten. Insbesondere die aus Kaltwasserfischen gewonnene Gelatine zeichnet sich durch eine relativ niedrige Gelierungstemperatur aus, d.h. wässrige Lösungen von (unvernetzter) Fischgelatine bleiben bei niedrigeren Temperaturen flüssiger als beispielsweise Lösungen von Schweineschwartengelatine gleicher Konzentration. Diese Tatsache erlaubt es, gelöste Fischgelatine bei Raumtemperatur oder sogar gekühlt bereitzustellen, was die Handhabung gegenüber einer Bereitstellung bei erhöhten Temperaturen von bis zu 37°C vereinfacht.

Gelatinearten bzw. Gelatinematerialien verschiedener Herkunft und/oder verschiedenen Typs lassen sich auch in einer Mischung als Gelatine erfindungsgemäß einsetzen, um die Eigenschaften der erfindungsgemäßen Zusammensetzung an die jeweilige Applikation noch besser anzupassen.

Um die Bioverträglichkeit der therapeutischen Zusammensetzung weiter zu verbessern, wird bevorzugt eine Gelatine mit einem besonders geringen Gehalt an Endotoxinen eingesetzt. Bei Endotoxinen handelt sich um Stoffwechselprodukte oder Bruchstücke von Mikroorganismen, welche in dem tierischen Rohmaterial vorkommen.

Der Endotoxingehalt von Gelatine wird in internationalen Einheiten pro Gramm (I.E./g) angegeben und gemäß dem LAL-Test bestimmt, dessen Durchführung in der vierten Ausgabe des Europäischen Arzneibuches (Ph. Eur. 4) beschrieben ist.

Um den Gehalt an Endotoxinen möglichst gering zu halten, ist es vorteilhaft, die Mikroorganismen möglichst frühzeitig im Zuge der Gelatineherstellung abzutöten. Ferner sollten entsprechende Hygienestandards beim Herstellungsprozess eingehalten werden.

Somit kann der Endotoxingehalt von Gelatine durch bestimmte Maßnahmen beim Herstellungsprozess drastisch gesenkt werden. Zu diesen Maßnahmen zählen in erster Linie die Verwendung frischer Rohmaterialien (z.B. Schweineschwarte) unter Vermeidung von Lagerzeiten, die sorgfältige Reinigung der gesamten Produktionsanlage unmittelbar vor Beginn der Gelatineherstellung sowie gegebenenfalls das Auswechseln von Ionenaustauschern und Filtersystemen in der Produktionsanlage.

Die im Rahmen der vorliegenden Erfindung eingesetzte Gelatine weist bevorzugt einen Endotoxingehalt von 1.200 I.E./g oder weniger, noch mehr bevorzugt von 200 I.E/g oder weniger auf. Optimalerweise liegt der Endotoxingehalt bei 50 I.E./g oder weniger, jeweils gemäß dem LAL-Test bestimmt. Im Vergleich hierzu weisen manche handelsübliche Gelatinen Endotoxingehalte von 20.000 I.E./g und mehr auf.

Bei Gelatine, die durch Extraktion von kollagenhaltigen Rohmaterialien gewonnen wird, handelt es sich, wie bereits angesprochen, um ein wasserlösliches Produkt, das insbesondere bei den für die Verabreichung geeigneten Temperaturen, d.h. 37°C oder weniger, in Lösung gebracht werden kann. Diese gelöste Form ist für die Verabreichung insofern besonders vorteilhaft, als dass die Gelatinelösung z.B. in den Nucleus pulposus einer Bandscheibe oder in ein anderes geschädigtes Gewebe, wie z.B. in Knorpel- oder Knochendefekte, injiziert werden kann. Um die Gelatine nach der Verabreichung, d.h. im Zielbereich des Körpers, in ein Gelatinegel zu überführen, erfolgt erfindungsgemäß eine Vernetzung der Gelatine.

Es sind verschiedene Arten von Vernetzungsmitteln bekannt, die die Gelatine durch inter- und/oder intramolekulare Verknüpfungen in ein Gelatinegel überführen, welches bei Temperaturen von 37°C oder weniger unlöslich ist. Bei diesen Verknüpfungen zwischen den Gelatinemolekülen kann es sich sowohl um kovalente Bindungen handeln als auch um eine Komplexbildung, die beispielsweise auf ionischen Wechselwirkungen, Wasserstoffbrücken oder Vander-Waals-Kräften beruht.

Vor dem Hintergrund der physiologischen Verträglichkeit wird bei der vorliegenden Erfindung auf ein enzymatisches Vernetzungsmittel zurückgegriffen: die Verwendung von Transglutaminase. Dieses Enzym, das in Tieren, Pflanzen und Bakterien vorkommt, katalysiert die Hydrolyse der Amidbindung von Glutaminresten und die Verknüpfung der dabei entstehenden freien Acylgruppe mit anderen Aminogruppen. Bei Proteinen, insbesondere bei der Gelatine, katalysiert die Transglutaminase somit in erster Linie eine Verknüpfung von Glutaminresten mit den ε-Aminogruppen von Lysinresten, d.h. die Bildung sowohl inter- als auch intramolekularer kovalenter Bindungen. Transglutaminase ist als natürliches Enzym physiologisch unbedenklich, sofern sie in entsprechend gereinigter Form zum Einsatz kommt.

Bevorzugt werden im Rahmen der Erfindung Transglutaminasen_{.}bakterieller Herkunft eingesetzt, die in hoher Qualität und Reinheit erhältlich sind. Es kann aber auch humane Transglutaminase eingesetzt werden, die insbesondere durch rekombinante Genexpression hergestellt werden kann.

Bevorzugt wird die Transglutaminase in immobilisierter Form auf einem Trägermaterial eingesetzt. Dadurch wird eine gleichmäßigere Verteilung der Enzymmoleküle in der Zusammensetzung möglich, sodass eine höhere Aktivität bei gleicher Menge an Enzym erreicht werden kann. Bevorzugte Trägermaterialien für Transglutaminase sind Oligosaccharide.

Erfindungsgemäß erfolgt die Vernetzung der Gelatine im Zielbereich des Körpers, d.h. die Gelatine und das Vernetzungsmittel sollten erst nach, während oder unmittelbar vor der Verabreichung unter Bedingungen miteinander in Kontakt kommen, die das Ablaufen der Vernetzungsreaktion ermöglichen. Um dies zu gewährleisten, sind verschiedene Formen der Bereitstellung und der Verabreichung der Gelatine und des Vernetzungsmittels denkbar. Die oben bereits erwähnten grundlegenden Alternativen (i) und (ii) sollen im Folgenden näher beschrieben werden.

Gemäß der Variante (i) der Erfindung erfolgt die Anwendung der Zusammensetzung dergestalt, dass die Gelatine und das Vernetzungsmittel zur Bildung einer vernetzenden therapeutischen Zusammensetzung gemischt werden und diese in den Zielbereich verabreicht wird. Bevorzugt handelt es sich bei einer solchen Zusammensetzung um eine wässrige Lösung, die das Vernetzungsmittel und die Gelatine gelöst enthält.

Bei dieser Vorgehensweise wird sichergestellt, dass eine homogene Verteilung beider Komponenten in der Lösung vorliegt. Eine solche Lösung kann auch auf einfache Weise verabreicht werden, insbesondere durch einfaches Auftragen auf den Zielbereich oder durch Injektion. Allerdings sollte eine solche Lösung in der Regel erst unmittelbar vor der Verabreichung hergestellt werden, um zu vermeiden, dass die Vernetzungsreaktion vor dem Erreichen des Zielbereiches bereits zu weit fortgeschritten ist und die Viskosität der Lösung z.B. für eine Injektion zu hoch wird. Je nach Art der Gelatine und des Vernetzungsmittels ist aber auch denkbar, dass eine die beiden Komponenten enthaltende Lösung einige Zeit gelagert werden kann, insbesondere bei niedrigen Temperaturen, ohne das die Vernetzungsreaktion bereits in einem die Verabreichung beeinträchtigenden Ausmaß abläuft.

Bevorzugt wird die wässrige Lösung durch Auflösen einer Feststoffmischung, welche die Gelatine und das Vernetzungsmittel vorzugsweise in lyophilisierter Form umfasst, hergestellt.

Das Bereitstellen von Gelatine und Vernetzungsmittel in dieser festen Form, in der die enzymatische Reaktion nicht ablaufen kann, hat den Vorteil, dass die Mischung eine relativ hohe Lagerstabilität aufweist. Gleichzeitig ist die Handhabung für den behandelnden Arzt einfach, da er nur eine einzige Feststoffmischung in einem flüssigen Medium, das gegebenenfalls die zu verabreichenden Zellen und/oder andere Wirkstoffe bereits enthält, auflösen muss.

Das Auflösen der Feststoffmischung sollte unmittelbar vor der Verabreichung der wässrigen Lösung erfolgen, d.h. insbesondere weniger als 10 Minuten, bevorzugt weniger als 5 Minuten vorher, bezogen auf eine in dem jeweiligen Zielbereich vorgegebene Temperatur.

Durch das Vorliegen der Gelatine in lyophilisierter Form wird deren Auflösung auch bei niedrigeren Temperaturen deutlich verbessert. Dies ist von Bedeutung, weil bei vielen bevorzugten Anwendungen der therapeutischen Zusammensetzungen gleichzeitig Zellen verabreicht werden, die gegenüber Temperaturen oberhalb von 37°C empfindlich sind. Das Auflösen der Feststoffmischung erfolgt deshalb vorzugsweise bei einer Temperatur von 37°C oder weniger. Lyophilisierte Gelatine ist bei diesen Temperaturen, insbesondere bei Raumtemperatur, gut löslich, da sie zumindest überwiegend in amorpher Form vorliegt.

Im Hinblick auf die Geschwindigkeit der Bildung des Gelatinegels sowie dessen Festigkeit ist die eingesetzte Menge an Vernetzungsmittel im Verhältnis zu der Menge an Gelatine von entscheidender Bedeutung. In der oben beschriebenen Mischung sind bevorzugt 0,6 bis 80 Units Transglutaminase pro Gramm Gelatine enthalten, weiter bevorzugt 5 bis 40 Units/g. Auf die Kinetik der Gelbildung, die sich unter anderem aus der Wahl dieses Verhältnisses ergibt, wird weiter unten im Detail eingegangen.

Im Hinblick auf die erste Variante (i) betrifft die vorliegende Erfindung somit auch eine Feststoffmischung, die Gelatine und Transglutaminase vorzugsweise in lyophilisierter Form umfasst.

Bei der oben genannten Variante (ii) der Erfindung erfolgt die Anwendung der Zusammensetzung dergestalt, dass die Gelatine und das Vernetzungsmittel in voneinander getrennter Form bereitgestellt und gleichzeitig oder nacheinander unter Bildung der vernetzenden therapeutischen Zusammensetzung verabreicht werden. Dabei kann eine Durchmischung der beiden Komponenten zu verschiedenen Zeitpunkten erfolgen, wie im Folgenden beschrieben wird.

Eine bevorzugte Form der Bereitstellung besteht darin, dass sowohl die Gelatine als auch das Vernetzungsmittel in Form von separaten wässrigen Lösungen bereitgestellt werden. Diese können dann vom behandelnden Arzt gemischt und in Form einer einzigen Lösung verabreicht werden, wie dies bereits oben beschrieben wurde. Das Mischen sollte dabei weniger als 10 Minuten, bevorzugt weniger als 5 Minuten vor der Verabreichung erfolgen.

Um einen zu frühen Beginn der Vernetzungsreaktion mit größerer Sicherheit auszuschließen, ist es jedoch bevorzugt, wenn die Gelatinelösung und die Vernetzungsmittellösung nicht vor, sondern erst während oder nach der Verabreichung miteinander in Kontakt kommen. Dies kann insbesondere durch eine gleichzeitige Verabreichung der beiden (getrennten) Lösungen erreicht werden.

Je nach Art der eingesetzten Mittel zur Verabreichung der Lösungen (z.B. eine oder mehrere Injektionskanülen oder andere Applikatoren) kann dabei die Durchmischung der gleichzeitig verabreichten Lösungen vor, beim oder nach dem Erreichen des Zielbereichs erfolgen. Eine möglichst frühzeitige Durchmischung, d.h. vor dem Erreichen des Zielbereichs, ist jedoch vorteilhaft, um eine hohe Homogenität der im Zielbereich ankommenden Lösung und damit die Ausbildung eines gleichmäßig vernetzten Gelatinegels zu gewährleisten.

Bei einer bevorzugten Ausführungsform der Erfindung erfolgt eine gleichzeitige Verabreichung der Gelatinelösung und der Vernetzungsmittellösung durch Injektion beider Lösungen mit einer Mehrkammerapplikationsvorrichtung, beispielsweise einer Doppelkammerspritze. Dabei befinden sich die Gelatine- und die Vernetzungsmittellösung in getrennten Kammern der Applikationsvorrichtung und werden beispielsweise durch eine gemeinsame Injektionskanüle an den gewünschten Zielbereich, beispielsweise den Nucleus pulposus, bereits vermischt verabreicht. Das Durchmischen der beiden Lösungen erfolgt somit bei der Verabreichung, beispielsweise beim Eintritt in die Kanüle. Um eine möglichst intensive Durchmischung zu bewirken, ist es bevorzugt, wenn die Mehrkammerapplikationsvorrichtung ein Mischelement umfasst. Dabei kann es sich insbesondere um eine geometrische Struktur (statischer Mischer) im Fließweg der Kanüle handeln, an der eine Durchmischung, insbesondere eine Verwirbelung der beiden Lösungen erfolgt.

Im Hinblick auf die zweite Variante (ii) betrifft die vorliegende Erfindung somit auch eine Mehrkammerapplikationsvorrichtung, welche eine wässrige Gelatinelösung und eine wässrige Vernetzungsmittellösung in getrennten Kammern enthält.

Alternativ ist es auch möglich, die wässrige Gelatinelösung und die wässrige Vernetzungsmittellösung nacheinander in den Zielbereich zu verabreichen und dort zu vermischen. Auch in diesem Fall ist sichergestellt, dass die Vernetzung der Gelatine erst im Zielbereich stattfindet.

Bei einer weiteren bevorzugten Ausführungsform der Erfindung wird einerseits eine wässrige Gelatinelösung bereitgestellt sowie ein Vernetzungsmittel in fester Form. Diese Variante ist insbesondere im Fall von enzymatischen Vernetzungsmitteln wie Transglutaminase geeignet, deren Haltbarkeit in dieser Form in der Regel höher ist als in Lösung. Das Enzym kann insbesondere in Form eines lyophilisierten Pulvers bereitgestellt werden, das dann vor dem Verabreichen in die Gelatinelösung zudosiert und nachfolgend aufgelöst wird.

Bei den Anwendungen der erfindungsgemäßen therapeutischen Zusammensetzung, die weiter unten im Detail erläutert werden, erfolgt gleichzeitig eine Verabreichung von lebenden Zellen, wobei diese bevorzugt in der wässrigen Gelatinelösung enthalten sind. Aufgrund der Temperaturempfindlichkeit dieser Zellen ist es bevorzugt, dass die Verabreichung der wässrigen Gelatinelösung bei einer Temperatur von 37°C oder weniger erfolgt. Die Herstellung der Gelatinelösung kann jedoch auch bei höheren Temperaturen, z.B. bei 60°C, erfolgen, wobei die Zellen dann erst nach dem Abkühlen auf 37°C oder weniger zugesetzt werden. Wird die Lösung dann bei Raumtemperatur oder unter Kühlung gelagert, kann die Gelatine zwar gelieren und sich verfestigen, sie kann dann aber durch Erwärmen auf bis zu 37°C unmittelbar vor der Verabreichung wieder in Lösung gebracht werden.

Die Konzentration der verabreichten Gelatinelösung ist bevorzugt so gewählt, dass die Gelatinekonzentration in der Zusammensetzung 5 bis 20 Gew.% beträgt. Es wurde gefunden, dass niedrigere Gelatinekonzentrationen in der Regel nicht gut zu vernetzten Gelatinegelen mit einer ausreichenden Festigkeit führen, wohingegen Konzentrationen von mehr als 20 Gew.% zum Teil nachteilig in Bezug auf die Lebensfähigkeit der Zellen sein können.

Im Fall von Transglutaminase ist deren Menge und Konzentration in einer Transglutaminaselösung vorzugsweise so gewählt, dass sich, wie bereits im Zusammenhang mit der Variante (i) beschrieben, eine Menge von 0,6 bis 80 Units Transglutaminase pro Gramm Gelatine in der Zusammensetzung ergibt. Weiter bevorzugt ist ein Verhältnis von 5 bis 40 Units/g. Dabei kann das Volumen der Transglutaminaselösung in der Regel deutlich kleiner gewählt werden als das der Gelatinelösung, so dass letztere durch das Mischen mit der Transglutaminaselösung nicht wesentlich verdünnt wird.

Die Geschwindigkeit der Vernetzungsreaktion sowie die Festigkeit des gebildeten Gelatinegels hängen in hohem Maße von der Gelatinekonzentration in der Zusammensetzung und von dem Verhältnis zwischen Gelatine und Vernetzungsmittel ab. Diese Parameter können innerhalb der oben genannten bevorzugten Bereiche variiert werden, um den Einfluss weiterer Faktoren auszugleichen.

Solche Faktoren sind z.B. die Art der eingesetzten Gelatine, insbesondere deren Viskosität und mittleres Molekulargewicht, sowie im Fall von Transglutaminase auch deren Art und Herkunft.

Die Kinetik und das Ausmaß der Vernetzungsreaktion können durch verschiedene physikalische Parameter beschrieben werden. Um diese zu messen, wird die Bildung des Gelatinegels, wie sie bei der therapeutischen Anwendung *in vivo* abläuft, durch eine entsprechende Reaktion *in vitro* nachgestellt. Der Beginn der Vernetzungsreaktion ist dabei jeweils durch den Zeitpunkt definiert, in dem die Gelatine und das Vernetzungsmittel in einer wässrigen Lösung miteinander in Kontakt kommen.

Die Geschwindigkeit der Bildung des vernetzten Gelatinegels lässt sich insbesondere durch die Angabe des sogenannten Gelpunktes charakterisieren. Der Gelpunkt ist dabei als derjenige Zeitpunkt nach dem Beginn der Vernetzungsreaktion definiert, an dem der Speichermodul G' und der Verlustmodul G" des Gelatinegels gleich groß sind (siehe auch T. Metzger, Das Rheologie-Handbuch, Verlag Vincentz, 2000, Seite 173 ff).

Bei einer unvernetzten, flüssigen Gelatinelösung liegt G' deutlich unterhalb von G". Im Verlauf der Vernetzungsreaktion, d.h. mit zunehmender Gelbildung, steigen sowohl der Speicher- als auch der Verlustmodul an, wobei G' stärker ansteigt als G". Der oben genannte Gelpunkt lässt sich somit aus dem Schnittpunkt der beiden Kurven in einem Diagramm, in dem G' und G" über der Zeit aufgetragen sind, ermitteln. Experimentell lässt sich der Gelpunkt auch als der Zeitpunkt ermitteln, an dem im Verlauf der Vernetzungsreaktion erstmals eine Gelfestigkeit (siehe unten) gemessen werden kann.

Für die Verwendung gemäß der vorliegenden Erfindung ist es bevorzugt, wenn der Gelpunkt des vernetzten Gelatinegels 5 bis 180 Minuten nach Beginn der Vernetzungsreaktion erreicht wird, besonders bevorzugt 10 bis 60 Minuten und am meisten bevorzugt bis zu 25 Minuten nach Beginn der Vernetzungsreaktion. Die vorgenannten bevorzugten Zeitvorgaben verstehen sich bezogen auf eine in dem jeweiligen Zielbereich vorgegebene Temperatur. Bei einer schnelleren Gelbildung besteht die Gefahr, dass die therapeutische Zusammensetzung ihre Fließfähigkeit zu früh verliert, was insbesondere dazu führen kann, dass dem behandelnden Arzt nicht genügend Zeit für die Verabreichung zur Verfügung steht oder dass die Gelbildung zu weit fortschreitet, bevor sich die Zusammensetzung am jeweiligen Zielort gleichmäßig verteilt hat. Eine zu langsame Gelbildung, d.h. ein zu später Gelpunkt, hat wiederum den Nachteil, dass die betroffene Körperstelle des Patienten, z.B. die Wirbelsäule im Fall der Bandscheibenregeneration, für eine unzumutbar lange Zeit fixiert werden muss.

Im Hinblick auf die mechanischen Eigenschaften des vernetzten Gelatinegels ist es bevorzugt, wenn dieses eine Gelfestigkeit von 100 g oder mehr aufweist, gemessen mit einem Stempel mit einem Durchmesser von 12,7 mm bei einer Eindringtiefe von 4 mm. Diese Angaben beziehen sich auf das Eindrücken eines kreisförmigen Stempels mit einem Durchmesser von 12,7 mm in das Gelatinegel senkrecht zu dessen Oberfläche, wobei der Stempel aus Polymethylmethacrylat besteht und eine polierte Oberfläche aufweist (siehe "Standardised Methods for the Testing of Edible Gelatine", Gelatine Monograph, Juni 2005, GME). Bei einer Gelfestigkeit von 100 g ist die diesem Gewicht entsprechende Kraft erforderlich, d.h. 0,981 N, um den Stempel 4 mm tief in das Gelatinegel einzudrücken.

Es wurde bereits angesprochen, dass neben anderen Faktoren auch die Viskosität der eingesetzten Gelatine einen Einfluss auf die Gelbildung ausübt, wobei eine höhere Viskosität in der Regel mit einer schnelleren Gelbildung einhergeht. Unter der Viskosität der Gelatine ist in diesem Zusammenhang die Viskosität einer 6,7 Gew.%igen Standardlösung der Gelatine in Wasser bei 60°C zu verstehen. Vorzugsweise beträgt die Viskosität der im Rahmen der vorliegenden Erfindung eingesetzten Gelatine 7 mPa·s oder mehr.

Die Viskosität von Gelatine ist sowohl von deren Herkunft als auch vom jeweiligen Herstellungsverfahren abhängig und kann durch bestimmte Maßnahmen weiter beeinflusst werden.

Bei einer bevorzugten Ausführungsform der Erfindung wird eine Gelatine eingesetzt, die zuvor einer thermischen Vorbehandlung bei vermindertem Druck unterworfen wurde. Durch eine solche Vorbehandlung lässt sich die Viskosität der Gelatine steigern, wobei dieser Effekt in erster Linie auf eine thermische Wasserabspaltung innerhalb der Gelatinemoleküle zurückzuführen ist.

Die thermische Vorbehandlung wird bevorzugt bei Temperaturen von 80 bis 160°C durchgeführt, da unterhalb von 80°C die beobachteten Effekte relativ schwach ausgeprägt sind und oberhalb von 160°C eine unerwünschte Verfärbung der Gelatine auftreten kann. Am meisten bevorzugt sind Werte im Bereich von 90 bis 120°C.

Die Gelbildung ist ferner auch vom Molekulargewicht der Gelatine abhängig. Die Verwendung von Gelatine mit einem hohen mittleren Molekulargewicht, insbesondere von 140 kDa oder mehr, ist dabei bevorzugt, da in diesem Fall bereits durch eine geringere Anzahl an Vernetzungsstellen ein unlösliches Gelatinegel entsteht als bei einer Gelatine mit niedrigerem Molekulargewicht.

Alternativ oder zusätzlich zu einer gezielten Auswahl oder Modifikation der eingesetzten Gelatine können die Eigenschaften der erfindungsgemäßen therapeutischen Zusammensetzung auch dadurch beeinflusst werden, dass zwei oder mehr Gelatinen mit unterschiedlichen Viskositäten und/oder Bloom-Werten miteinander gemischt werden. Beispielsweise kann durch verschiedene Mischungsverhältnisse von hochviskoser Knochengelatine mit einer niedrigviskosen Fischgelatine die Geschwindigkeit der Gelbildung über einen weiten Bereich variiert werden.

Bei einer weiteren bevorzugten Ausführungsform der Erfindung wird für die Herstellung der therapeutischen Zusammensetzung eine partiell vernetzte Gelatine eingesetzt, d.h. die Gelatine wird vor der erfindungsgemäßen Verabreichung bereits einem ersten (partiellen) Vernetzungsschritt unterzogen. Die partiell vernetzte Gelatine kann, wie oben beschrieben, mit dem Vernetzungsmittel gemischt oder mit diesem gleichzeitig oder aufeinander folgend verabreicht werden, wobei die Bildung des vernetzten Gelatinegels im Zielbereich dann einen zweiten Vernetzungsschritt darstellt.

Durch die Verwendung von partiell vernetzter Gelatine kann einerseits die Viskosität der zu verabreichenden Gelatinelösung deutlich erhöht werden, was mit den oben erwähnten Vorteilen einhergeht. Des Weiteren kann durch diese Maßnahme auch eine wesentlich schnellere Gelbildung erreicht werden, wobei Gelpunkte deutlich unterhalb von 5 Minuten, insbesondere im Bereich von wenigen Sekunden, realisiert werden können. Eine sehr schnelle Gelbildung, die fast unmittelbar nach der Verabreichung der therapeutischen Zusammensetzung einsetzt, kann bei bestimmten Anwendungen vorteilhaft sein.

Damit die Lösung der partiell vernetzten Gelatine zwar hochviskos, aber unter den Applikationsbedingungen immer noch fließfähig ist, sollte der Grad der partiellen Vernetzung nicht zu hoch sein. Dieser kann durch die Bedingungen, unter denen die partiell vernetzte Gelatine hergestellt wird, kontrolliert werden, insbesondere durch die Gelatinekonzentration, die Menge des Vernetzungsmittels und die Dauer der partiellen Vernetzungsreaktion. Bevorzugt ist die eingesetzte Gelatine unter Verwendung von Transglutaminase partiell vernetzt. Neben den bereits oben erwähnten Vorteilen bietet die Verwendung von Transglutaminase die Möglichkeit, die partielle Vernetzungsreaktion durch die Inaktivierung des Enzyms nach einer definierten Reaktionszeit abzubrechen, insbesondere durch eine thermische Denaturierung oder ein Oxidationsmittel wie z.B. Wasserstoffperoxid.

Wenn die partielle Vernetzung der Gelatine mittels Transglutaminase erfolgt, so können hierfür deutlich geringere Mengen an Transglutaminase im Verhältnis zur Gelatine eingesetzt werden, als dies im Rahmen der Verabreichung der vernetzenden therapeutischen Zusammensetzung der Fall ist. Vorzugsweise ist die Gelatine unter Verwendung von weniger als 10 Units Transglutaminase pro Gramm Gelatine partiell vernetzt, insbesondere unter Verwendung von 1 bis 3 Units Transglutaminase pro Gramm Gelatine.

Die erfindungsgemäße therapeutische Zusammensetzung ist, wie eingangs beschrieben, zur Bildung eines vernetzten Gelatinegels als Zellmatrix bestimmt, d.h. als eine feste Matrix, die das Wachstum und/oder die Differenzierung von Zellen unterstützt.

Die therapeutische Zusammensetzung umfasst lebende Zellen oder eine Kombination von Zellen und Wachstumsfaktoren, die insbesondere mit dem Ziel der biologischen Regeneration oder Rekonstruktion von Gewebe an den jeweiligen Zielbereich des Körpers verabreicht werden. Auf einige dieser Anwendungen soll im Folgenden näher eingegangen werden.

Eine bevorzugte Ausführungsform der Erfindung betrifft die Verwendung von Gelatine und eines Vernetzungsmittels zur Herstellung einer vernetzenden therapeutischen Zusammensetzung zur Regeneration von Bandscheibenschäden.

Unter Bandscheibenschäden ist dabei jegliche Degeneration oder Beeinträchtigung der natürlichen Funktion des Bandscheibengewebes zu verstehen, insbesondere aber im Bereich des Nucleus pulposus (Gallertkern) oder des Annulus fibrosus (schützender Faserring der Bandscheibe). Mit zunehmendem Alter kann es in Abhängigkeit von anderen Einflussfaktoren zu einer Abnahme der Vitalität der Zellen im Gallertkern und/oder im Faserring der Bandscheiben kommen, was dann dazu führt, dass von diesen Zellen nur eingeschränkt oder überhaupt keine extrazelluläre Matrix mehr produziert wird. Diese extrazelluläre Matrix ist jedoch von entscheidender Bedeutung für die Elastizität und damit die Pufferwirkung der Bandscheibe, da sie in der Lage ist, große Mengen an Wasser zu binden. Als Folge dieser Degeneration kann, wie bereits eingangs erwähnt, ein erheblicher Funktionsverlust der Bandscheibe mit chronischen Schmerzen oder durch Ausfaserung bzw. Beschädigung des Annulus fibrosus ein Bandscheibenvorfall entstehen.

Bei der biologischen Bandscheibenrekonstruktion wird versucht, solchen Schädigungen entgegenzuwirken, indem Zellen und/oder Wachstumsfaktoren in die verschiedenen Strukturen der Bandscheibe eingebracht werden, um dort neue extrazelluläre Matrix zu synthetisieren. Hierfür ist die Verwendung von Gelatine und eines Vernetzungsmittels gemäß vorliegender Erfindung besonders geeignet, wobei bei dieser Ausführungsform der Ziel bereich der Nucleus pulposus und/oder der Annulus fibrosus der Bandscheibe ist.

Gemäß der Erfindung werden dabei Wachstumsfaktoren und/oder Zellen, die gegebenenfalls *in vitro* vorkultiviert wurden, gemeinsam mit der Gelatine und dem Vernetzungsmittel in den Zielbereich der Bandscheibe verabreicht. Nach Verteilung der zunächst flüssigen Zusammensetzung in den verschiedenen Strukturen der Bandscheibe bildet sich innerhalb kurzer Zeit nach ihrer Verabreichung, d.h. bevorzugt innerhalb weniger Minuten, das vernetzte Gelatinegel. Dieses Gel sorgt einerseits dafür, dass die Zellen und/oder Wachstumsfaktoren in einer gleichmäßigen Verteilung in der Bandscheibe fixiert werden, und gleichzeitig verhindert es, dass die Zellen und/oder Wachstumsfaktoren aufgrund eines auf die Bandscheibe ausgeübten Druckes nennenswert wieder aus der Bandscheibe herausgedrückt werden können, da die vernetzte Gelatine die schalenförmig aufgebauten Strukturen des Annulus fibrosus und die Eintrittspforte der Injektion verklebt und damit verschließt. Eine Fixierung bzw. Ruhigstellung des Patienten ist daher nur für eine verhältnismäßig kurze Zeit erforderlich, d.h. bis die Bildung des Gelatinegels im Wesentlichen abgeschlossen ist, was beispielsweise innerhalb von 5 bis 180 Minuten geschehen kann.

Die Anwendung der therapeutischen Zusammensetzung kann insbesondere durch Verabreichung einer wässrigen Lösung erfolgen, die die Gelatine und das Vernetzungsmittel enthält, oder durch die gleichzeitige Verabreichung einer wässrigen Gelatinelösung und einer wässrigen Vernetzungsmittellösung und das Vermischen derselben.

Diese und weitere Varianten der Bereitstellung und Verabreichung wurden bereits oben beschrieben. In beiden Fällen kann die Verabreichung minimalinvasiv mittels Injektion erfolgen, wobei zur Durchführung der zweiten Variante, wie oben beschrieben, bevorzugt eine Mehrkammerinjektionsvorrichtung zum Einsatz kommt.

Die zu verabreichenden Zellen und/oder Wachstumsfaktoren sind dabei bevorzugt in der wässrigen Lösung, die die Gelatine und das Vernetzungsmittel enthält, bzw. in der wässrigen Gelatinelösung enthalten.

In dem Fall, dass die Verabreichung einer einzelnen Lösung erfolgt, kann die Bereitstellung der Wachstumsfaktoren und/oder der Zellen insbesondere in Form einer Lösung oder Zellsuspension in einem geeigneten flüssigen Medium erfolgen. Bei einer bevorzugten Ausführungsform der Erfindung wird dann durch den behandelnden Arzt unmittelbar vor der Verabreichung eine Feststoffmischung aus Gelatine und Transglutaminase in lyophilisierter Form in dieser Lösung bzw. Zellsuspension aufgelöst. Wie bereits beschrieben, liegt die Gelatine dabei in überwiegend amorpher Form vor, so dass sie bei einer Temperatur von 37°C oder weniger löslich ist.

Im zweiten Fall können die Wachstumsfaktoren, die Zellen oder eine Kombination von Zellen mit Wachstumsfaktoren dem Arzt bereits in der wässrigen Gelatinelösung zur Verfügung gestellt werden, im Idealfall bereits in einer auch mit der Vernetzungsmittellösung gefüllten Mehrkammerapplikationsvorrichtung, um so die Gefahr einer Kontamination der Wachstumsfaktoren und/oder der Zellen zu minimieren. Sofern die Gelatine während der Lagerung oder des Transportes geliert, kann sie durch Erwärmen der Mehrkammerapplikationsvorrichtung auf eine Temperatur von bis zu 37°C unmittelbar vor der Verabreichung wieder in Lösung gebracht werden. Die Anwendung der therapeutischen Zusammensetzung ist bei dieser Ausführungsform besonders einfach, da der behandelnde Arzt lediglich eine Injektionskanüle unter kontrollierten Bedingungen (z.B. mit Hilfe bildgebender Verfahren) an den gewünschten Zielbereich einführt und die vorgewärmte Mehrkammerapplikationsvorrichtung hieran anschließt.

Die im Rahmen der Bandscheibenrekonstruktion eingesetzten Zellen sind bevorzugt Bandscheibenzellen. Diese weisen eine hohe Ähnlichkeit zu Knorpelzellen (Chondrozyten) auf und werden daher auch als chondrogene Zellen bezeichnet. Als Alternative hierzu können aber auch mesenchymale Stammzellen zum Einsatz kommen. Diese Zellen können aus Knochenmark isoliert werden und weisen das Potenzial auf, in Richtung Chondrozyten oder Bandscheibenzellen zu differenzieren.

Die verwendeten Zellen können sowohl autologer Herkunft sein, d.h. sie wurden dem Patienten selbst entnommen, als auch allogener Herkunft, d.h. von einem Spender stammend.

Bei der Bereitstellung der zu verabreichenden Zellen wird vorteilhafterweise so vorgegangen, dass Zellen des betreffenden Typs aus dem Gewebe isoliert und *in vitro* vorkultiviert werden. Eine alternative Möglichkeit, die im Rahmen der vorliegenden Erfindung ebenfalls zum Einsatz kommen kann, besteht darin, zerkleinertes Gewebe, welches die gewünschten Zellen enthält, mit der therapeutischen Zusammensetzung zu verabreichen. Im Vergleich zu einer Anzucht der Zellen *in vitro* ist diese Methode sehr schnell und kostengünstig durchzuführen. Insbesondere wird dabei dem Patienten oder einem Spender ein Gewebestück mittels Biopsie entnommen und mechanisch zerkleinert, z.B. mit einem sterilen Skalpell oder einer Mühle. Die resultierenden Gewebestücke sollten ausreichen klein sein, um nach der Verabreichung eine Migration der lebenden Zellen aus dem Gewebe in das vernetzte Gelatinegel, d.h. die Zellmatrix, zu ermöglichen, vorzugsweise kleiner als 1 mm³. Vorzugsweise wird dann eine Suspension der Gewebefragmente hergestellt (z.B. 300 bis 500 mg Gewebe in 1 ml einer physiologischen Pufferlösung), die dann mit der wässrigen Gelatinelösung bzw. der die Gelatine und das Vernetzungsmittel enthaltenden Lösung gemischt werden kann.

Am Zielbereich, d.h. im oben beschriebenen Fall im Nucleus pulposus und/oder Annulus fibrosus der Bandscheibe, bildet das vernetzte Gelatinegel eine das Wachstum und/oder die Differenzierung der Zellen unterstützende Matrix. Eine wichtige Rolle spielt dabei auch die Tatsache, dass das Gelatinegel nach einer bestimmten Zeit abgebaut bzw. resorbiert wird und somit sukzessive durch die von den Zellen synthetisierte extrazelluläre Matrix ersetzt werden kann.

Um möglichst optimale Bedingungen für die Zellen bereitzustellen, ist es, wie bereits angedeutet, bevorzugt, wenn die therapeutische Zusammensetzung Wachstumsfaktoren oder andere protektiv und stimulierend wirkende Substanzen für die Zellen umfasst. Solche Faktoren sind im Rahmen der Bandscheibenregeneration beispielsweise Faktoren der TGF-β-Superfamilie (transforming growth factor beta), Insulin, Glukosamine, Chondroitinsulfat, Hyaluronsäure oder auch entzündungshemmende Wirkstoffe (wie IL-1-Rezeptorantagonisten) oder antibiotisch wirkende Substanzen. Durch solche Faktoren wird die Überlebensrate, die Proliferation und Differenzierung der Zellen sowie das Stoffwechselmilieu im Zielbereich positiv beeinflusst.

Generell ist in Bezug auf die Vitalität der eingesetzten Zellen darauf zu achten, dass die sie enthaltende wässrige Lösung physiologische Bedingungen aufweist, d.h. insbesondere einen geeigneten pH-Wert und Ionenstärke. Beispielsweise können bekannte Puffersysteme wie PBS-Puffer oder Hanks-Puffer eingesetzt werden. Dabei muss allerdings beachtet werden, dass durch die gelöste Gelatine die Osmolarität, d.h. die osmotisch wirksame Gesamtkonzentration der Lösung, erhöht wird. Um negative Effekte auf die Zellen zu vermeiden, sollte dies durch eine Absenkung der Konzentration anderer Komponenten des Puffersystems ausgeglichen werden.

Gegenstand der vorliegenden Erfindung ist in diesem Zusammenhang auch ein Verfahren zur Regeneration von Bandscheibenschäden, wobei das Verfahren die Verabreichung von Gelatine, Transglutaminase sowie Bandscheibenzellen und/oder Chondrocyten und/oder mesenchymalen Stammzellen und/ oder Wachstumsfaktoren in den Nucleus pulposus und/oder Annulus fibrosus der Bandscheibe getrennt oder bereits vor Erreichen des Zielbereiches gemischt umfasst.

In analoger Weise, wie dies im Zusammenhang mit Bandscheibenschäden beschrieben wurde, kann die therapeutische Zusammensetzung der vorliegenden Erfindung auch im Bereich des Meniskus oder des artikulären oder des nicht artikulären Knorpels angewendet werden. Insofern betrifft die Erfindung auch die Verwendung von Gelatine und einem Vernetzungsmittel zur Herstellung einer vernetzenden therapeutischen Zusammensetzung zur Regeneration von Meniskus- oder Knorpelschäden.

Neben der Bandscheibe verfügt auch Knorpelgewebe über eine äußerst geringe intrinsische Regenerationsfähigkeit. Im Falle von Schädigungen, z.B. bei verletzungsbedingten Knorpelläsionen in Folge eines Traumas, einer Osteochondrosis dissecans oder bei begrenzt degenerativen Knorpelschäden, werden daher autologe oder allogene Chondrozyten in den Defektbereich eingebracht, um dort neue extrazelluläre Matrix zu produzieren (autologe oder allogene Knorpelzelltransplantation).

Die Verabreichung dieser Zellen kann, insbesondere bei räumlich kleineren Defekten, vorteilhafterweise unter Anwendung der therapeutischen Zusammensetzung der vorliegenden Erfindung erfolgen, wie dies bereits im Zusammenhang mit der Bandscheibenregeneration beschrieben wurde. Bei den eingesetzten Zellen kann es sich dabei um Chondrozyten und/oder um mesenchymale Stammzellen handeln, wobei letztere über das Potenzial verfügen, in Richtung Chondrozyten zu differenzieren.

Diese und weitere Vorteile der vorliegenden Erfindung werden anhand der nachfolgenden Beispiele unter Bezugnahme auf die Figuren näher erläutert. Es zeigen:
- Fig. 1A bis 1D:: Diagramme, in denen die Gelfestigkeit und Klebrigkeit eines erfindungsgemäßen vernetzten Gelatinegels für Gelatinen mit verschiedenen Viskositäten in Abhängigkeit von der Re- aktionszeit aufgetragen sind; und.
- Fig. 2:: eine lichtmikroskopische Aufnahme eines erfindungsgemä- ßen vernetzten Gelatinegels mit darin eingebetteten Chon- drozyten.

### Beispiele

### Beispiel 1: Vernetzung von Gelatine mit Transglutaminase: Einfluss der Viskosität der Gelatine

Als Modellsystem für die medizinische Anwendung der erfindungsgemäßen therapeutischen Zusammensetzung wurde die Vernetzung von Gelatine mit dem enzymatischen Vernetzungsmittel Transglutaminase *in vitro* durchgeführt und die Kinetik der Bildung eines vernetzten Gelatinegels bestimmt.

### Herstellung einer Transglutaminase-Stammlösung

Für dieses und die nachfolgend beschriebenen Beispiele wurde eine rekombinante Transglutaminase aus humanen Keratinozyten eingesetzt.

Eine Stammlösung der Transglutaminase mit einer Konzentration von 30 Units/ml wurde hergestellt, indem die entsprechende Menge des Enzyms in destilliertem Wasser bei Raumtemperatur aufgelöst wurde. Die Lösung wurde sterilfiltriert, in Portionen von je 1,5 ml mit Hilfe von flüssigem Stickstoff eingefroren und bei ca. -18°C gelagert. Alternativ können die Portionen bei +4°C gelagert werden.

### Thermische Vorbehandlung von Gelatine bei vermindertem Druck

Für die Vernetzung mit Transglutaminase wurden Schweineschwartengelatinen mit unterschiedlichen Viskositäten gemäß der folgenden Tabelle 1 eingesetzt. Die Angabe der Viskosität bezieht sich dabei stets auf die Viskosität einer 6,7 Gew.%igen wässrigen Lösung der Gelatine bei 60°C.

**Tabelle 1**

| Bezeichnung | Viskosität (mPa·s) |
|---|---|
| Gelatine A | 3,73 |
| Gelatine B | 5,83 |
| Gelatine C | 7,62 |
| Gelatine D | 8,65 |

Die hochviskosen Gelatinen C und D wurden jeweils durch eine thermische Vorbehandlung von Gelatinen mit geringerer Viskosität hergestellt. Dabei wurde die Gelatine C durch thermische Vorbehandlung der Gelatine B erhalten sowie die Gelatine D durch thermische Vorbehandlung einer weiteren Schweineschwartengelatine mit einer Viskosität von 6,41 mPa·s.

Die thermische Vorbehandlung der Gelatine bei vermindertem Druck wurde so durchgeführt, dass jeweils ca. 700 g Gelatine in gemahlener Form mit Hilfe eines Rotationsverdampfers für 4 Stunden bei 105°C unter einem Vakuum von ca. 14 mbar gehalten wurden. Anschließend wurde die Gelatine über Nacht in einem geschlossenen Gefäß abkühlen gelassen.

### Durchführung der Vernetzungsreaktion

Für jede der vier Gelatinen A, B, C und D wurde eine 10 Gew.%ige Lösung der Gelatine in einer Mischung aus 30 Vol.% PBS-Puffer (pH 7,2) und 70 Vol.% destilliertem Wasser hergestellt. Hierfür wurde die Gelatine bei 60°C gelöst und die resultierende homogene Lösung auf 37°C temperiert.

Sämtliche Vernetzungsreaktionen wurden bei einer konstanten Temperatur von 37°C durchgeführt, um die bei der therapeutischen Anwendung herrschenden Bedingungen möglichst gut anzunähern. Für jeden Ansatz wurden 5 ml der 10 Gew.%igen Gelatinelösung in ein zylindrisches Gefäß mit einem Durchmesser von 3 cm gegeben, das mit Hilfe eines Aluminiumblockes auf 37 °C temperiert wurde. Die Vernetzungsreaktion wurde gestartet, indem 0,3 ml der Transglutaminase-Stammlösung (30 Units/ml) und 0,9 ml destilliertes Wasser, jeweils auf 37°C vorgewärmt, hinzugegeben wurden und die resultierende Reaktionsmischung sofort durchmischt wurde. Dies entspricht einer auf die Gelatine bezogenen Enzymmenge von 18 Units/g.

### Bestimmung der Gelfestigkeit in Abhängigkeit von der Reaktionszeit

Während des Verlaufs der Vernetzungsreaktion wurden in Abständen von 50 s mittels einer Kraft/Weg-Messvorrichtung vom Typ Zwick BZ 2.5/TN1S (Hersteller: Zwick GmbH & Co. KG, Ulm) die Gelfestigkeit und die Klebrigkeit der Reaktionsmischung bestimmt.

Die Bestimmung läuft so ab, dass bei jedem Messzyklus, d.h. alle 50 s, ein kreisförmiger Stempel mit einem Durchmesser von 12,7 mm senkrecht zur. Oberfläche der Reaktionsmischung 4 mm tief in diese eingetaucht bzw. eingedrückt und die hierfür erforderliche Kraft gemessen wird. Anschließend wird der Stempel, der eine polierte Polymethylmethacrylatoberfläche aufweist, wieder nach oben gezogen. Sofern bereits ein vernetztes Gelatinegel vorliegt, haftet dieses beim Herausziehen des Stempels an diesem an. Die erforderliche Kraft, um den Stempel soweit nach oben zu ziehen, dass sich das Gelatinegel ablöst, wird ebenfalls gemessen.

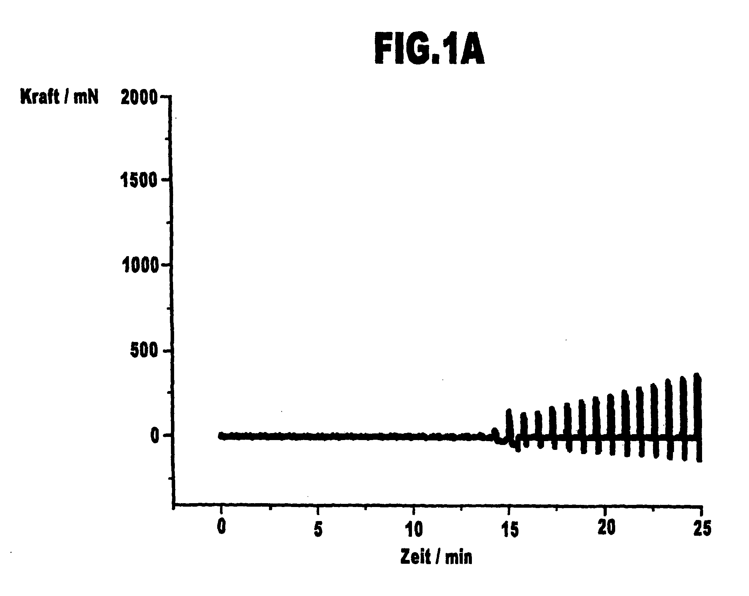

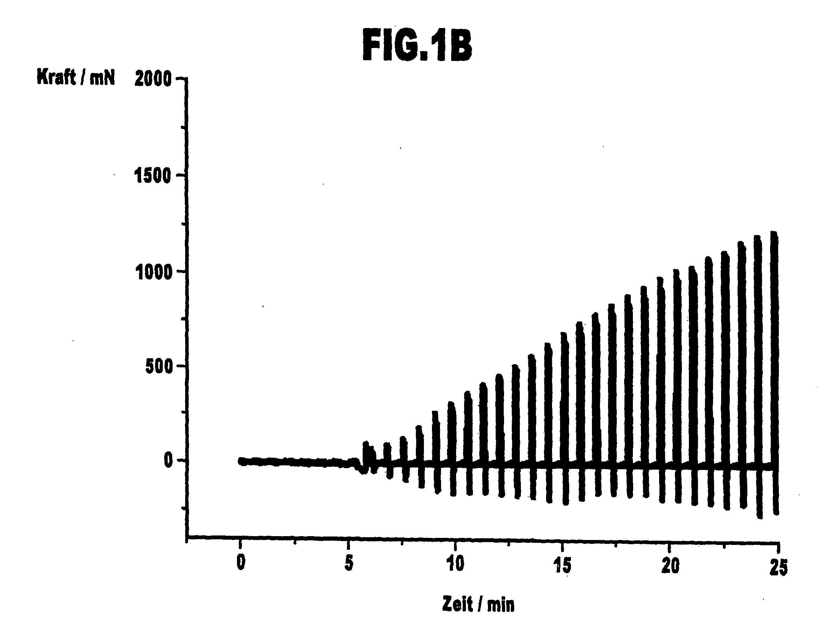

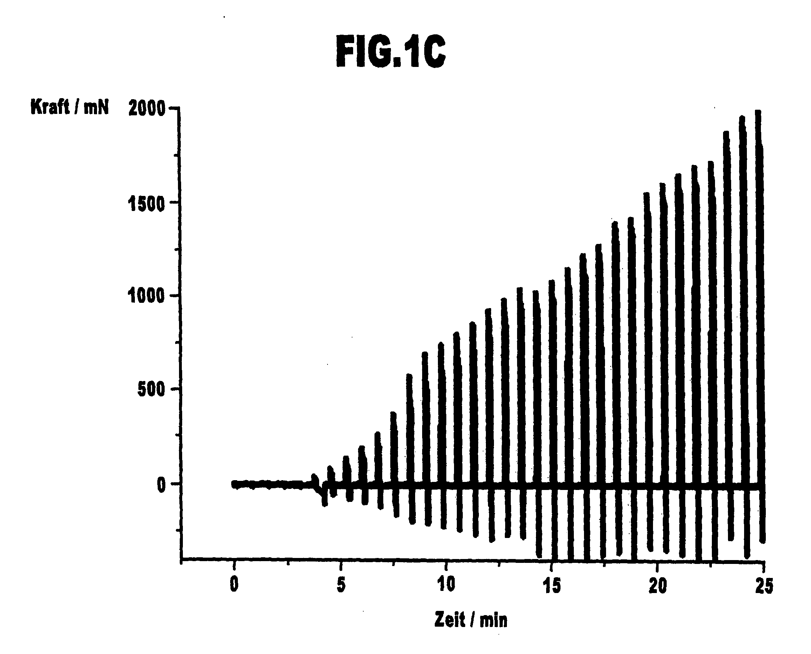

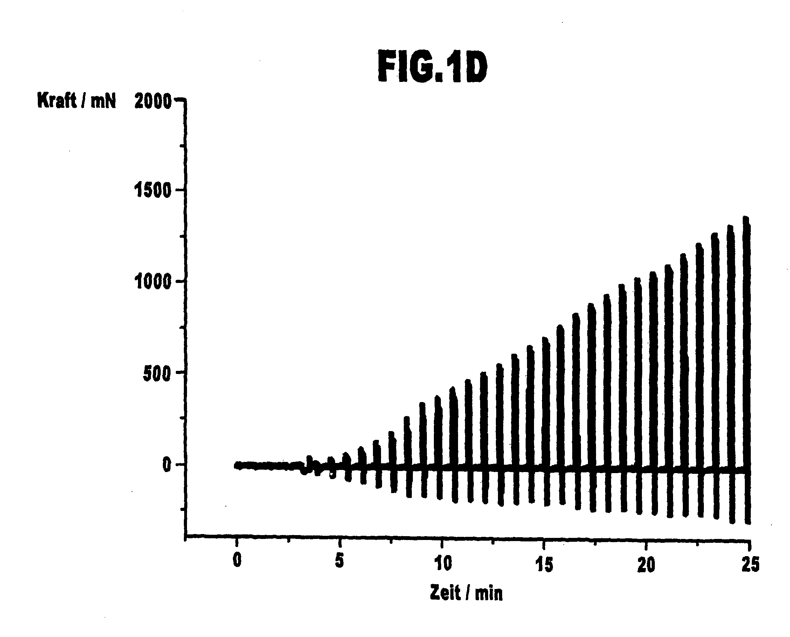

In den Figuren 1A bis 1D ist die gemessene Kraft in Abhängigkeit von der Reaktionszeit (Beginn der Vernetzungsreaktion bei 0 min) für die vier Ansätze mit den Gelatinen A, B, C bzw. D aufgetragen. Die positiven Kraftwerte geben die für das Eindrücken des Stempels erforderliche Kraft an, d.h. die Gelfestigkeit (981 mN entsprechen einer Gelfestigkeit von 100 g). Die negativen Kraftwerte geben die Klebrigkeit des Gelatinegels an, d.h. die zum Herausziehen des Stempels bis zur Ablösung des Gelatinegels erforderliche Kraft.

Während einer Anfangsphase der Reaktion liegen sowohl die Gelfestigkeit als auch die Klebrigkeit im Wesentlichen bei null, d.h. es liegt eine fließfähige Lösung vor. Bei der medizinischen Anwendung entspricht dies dem Zeitraum, in dem die Mischung an den Zielbereich des Körpers verabreicht werden kann. Der früheste Zeitpunkt, an dem eine merklich von null verschiedene Gelfestigkeit gemessen werden kann, ist der Gelpunkt. Zu diesem Zeitpunkt sind der Speichermodul G' und der Verlustmodul G" gleich groß.

Die verschiedenen Gelpunkte der Ansätze A bis D sind in der folgenden Tabelle 2 aufgeführt, wobei die Werte jeweils aus drei Experimenten gemittelt sind.

**Tabelle 2**

| Ansatz | Viskosität | Gelpunkt | Gelfestigkeit nach 10 min |
|---|---|---|---|
| Gelatine A | 3,73 mPa·s | 14 min | 281 mN/cm² |
| Gelatine B | 5,83 mPa·s | 5 min | 536 mN/cm² |
| Gelatine C | 7,62 mPa·s | 3,3 min | 837 mN/cm² |
| Gelatine D | 8,65 mPa·s | 2,3 min | nicht bestimmt |

Es wird deutlich, dass der Gelpunkt mit der Viskosität der eingesetzten Gelatine korreliert, d.h. bei einer hochviskosen Gelatine erfolgt die Gelbildung bei gleicher Menge an Vernetzungsmittel wesentlich schneller als bei einer niedrigviskosen. Auch die erzielte Gelfestigkeit ist vom Ausgangsmaterial abhängig, wie sich aus den Figuren 1A bis 1D ergibt: nach 25 min wurden bei den Gelatinen B, C und D Gelfestigkeiten von z.T. deutlich über 100 g erreicht, während bei der Gelatine A mit der niedrigsten Viskosität nur ca. 40 g erreicht wurden.

### Beispiel 2: Vernetzung von Gelatine mit Transglutaminase: Einfluss der Gelatinekonzentration

In diesem Beispiel wurde die thermisch vorbehandelte Gelatine C aus dem Beispiel 1 bei unterschliedlichen Gelatinekonzentrationen mit Transglutaminase vernetzt. Das Ansetzen der Reaktionsmischungen und die Messung der Gelfestigkeit erfolgten wie in Beispiel 1 beschrieben.

Die Konzentration der eingesetzten Gelatinelösungen, die Zusammensetzung der Reaktionsmischungen und die sich aus der Gelfestigkeitsmessung ergebenden Gelpunkte sind in der folgenden Tabelle 3 dargestellt.

**Tabelle 3**

| Ansatz | 2-1 | 2-2 | 2-3 |
|---|---|---|---|
| Konzentration der Gelatinelösung | 5 Gew.% | 8 Gew.% | 10 Gew.% |
| Gelatinelösung | 5,9 ml | 5,9 ml | 5 ml |
| Transglutaminase-Stammlösung | 0,2 ml | 0,3 ml | 0,3 ml |
| destilliertes Wasser | - | - | 0,9 ml |
| Transglutaminase pro Gramm Gelatine | 20,3 Units/g | 19,1 Units/g | 18,0 Units/g |
| Gelpunkt | 5,0 min | 4,0 min | 3,3 min |

Die Ergebnisse zeigen, dass durch eine Erhöhung der Gelatinekonzentration die Gelbildung beschleunigt werden kann. Bei den durchgeführten Versuchen ist dieser Trend deutlich erkennbar, obwohl bei den höheren Gelatinekonzentrationen die Menge an Vernetzungsmittel im Verhältnis zur Gelatine etwas geringer war.

Bei der medizinischen Anwendung der erfindungsgemäßen Zusammensetzung muss in diesem Zusammenhang beachtet werden, dass eine Erhöhung der Gelatinekonzentration auf Werte über ca. 20 Gew.% hinaus unter Umständen negative Effekte auf die Lebesfähigkeit von Zellen haben kann, die in der Gelatinelösung bereitgestellt werden.

### Beispiel 3: Vernetzung von Gelatine mit Transglutaminase: Einfluss der Menge an Vernetzungsmittel

In diesem Beispiel wurde die thermisch vorbehandelte Gelatine C aus dem Beispiel 1 mit unterschiedlichen Mengen an Transglutaminase vernetzt.

Eine 5 Gew.%ige Lösung der Gelatine C wurde hergestellt wie in Beispiel 1 beschrieben. Für jeden Ansatz wurden 5,9 ml dieser Lösung auf 37°C vorgewärmt und mit der in der nachfolgenden Tabelle 4 angegebenen Menge an Transglutaminase-Stammlösung (30 U/ml, auf 37°C vorgewärmt) versetzt, um die Vernetzungsreaktion zu starten. Die Bestimmung des Gelpunktes anhand der Gelfestigkeitsmessung erfolgte wie in Beispiel 1 beschrieben.

**Tabelle 4**

| Ansatz | 3-1 | 3-2 | 3-3 | 3-4 |
|---|---|---|---|---|
| Menge an Transglutaminase-Stammlösung | 0,1 ml | 0,2 ml | 0,3 ml | 0,5 ml |
| Transglutaminase pro Gramm Gelatine | 10,2 Units/g | 20,3 Units/g | 30,5 Units/g | 50,8 Units/g |
| Gelpunkt | 14,0 min | 5,0 min | 4,0 min | 3,0 min |

Wie aus den in der Tabelle 4 angegebenen Werten hervorgeht, kann die Geschwindigkeit der Bildung des vernetzten Gelatinegels auch über die Konzentration des Vernetzungsmittels, in diesem Fall der Transglutaminase, beeinflusst werden. Erwartungsgemäß führt eine höhere Menge an Vernetzungsmittel zu einer schnelleren Gelbildung.

### Beispiel 4: Vernetzung von Gelatine mit Transglutaminase in einem Zellkulturmedium

In diesem Beispiel wurde die Gelatine C aus dem Beispiel 1 in unterschiedlichen Konzentrationen mit unterschiedlichen Mengen an Transglutaminase in einem besonders gut für die Anzucht von Zellen geeigneten Puffersystem vernetzt. Dazu wurde eine wässrige Gelatinelösung in einer Mischung aus 80 Vol.% des Zellkulturmediums DMEM/F12 (Dulbecco's Modified Eagel Medium, BioWhittaker) und 20 Vol.% FB-Serum (Fötales Kälberserum, PAA Laboratories) hergestelt. Die Durchführung der Vernetzungsreaktion und die Messung der Gelfestigkeit erfolgten im Übrigen wie in Beispiel 1 beschrieben mit den jeweils in der Tabelle 5 angegebenen Mengenverhältnissen.

**Tabelle 5**

| Ansatz | 4-1 | 4-2 | 4-3 | 4-4 |
|---|---|---|---|---|
| Gelatinekonzentration | 12,7 Gew.% | 8 Gew.% | 8 Gew.% | 6 Gew.% |
| Menge an Gelatinelösung | 5,9 ml | 5,9 ml | 5,9 ml | 5,9 ml |
| Menge an Transglutaminase-Stammlösung | 0,3 ml | 0,3 ml | 0,2 ml | 0,2 ml |
| Transglutaminase pro Gramm Gelatine | 12,0 U/g | 19,1 U/g | 12,7 U/g | 16,9 U/g |
| Transglutaminase-Konzentration | 1,45 U/ml | 1,45 U/ml | 0,98 U/ml | 0,98 U/ml |
| Vergelung | 3,0 min | 5,0 min | 8,0 min | 11,0 min |
| Gelfestigkeit 10 min nach dem Gelpunkt | 1420 mN | 620 mN | 470 mN | 280 mN |

Die Ergebnisse zeigen, dass sich die Vernetzung von Gelatine mit Transglutaminase auch in einem Zellkulturmedium, welches für die Anzucht und Verabreichung von lebenden Zellen geeignet ist, problemlos durchführen lässt. Wie in den vorhergehenden Beispielen zeigt sich auch hier, dass durch die Wahl der Gelatine- und Vernetzungsmittelkonzentration die Geschwindigkeit der Gelbildung und die erzielte Gelfestigkeit über einen weiten Bereich variiert werden können.

### Beispiel 5: Vernetzung von nicht gelierender Gelatine mit Transglutaminase

In diesem Beispiel wurde eine unter den Bedingungen des Standard-Bloom-Tests nicht gelierende Gelatine eingesetzt. Diese aus Fischhaut gewonnene Gelatine ist folgendermaßen charakterisiert:

| | |
|---|---|
| Gelfestigkeit: | 0 g Bloom |
| Viskosität (6,7 Gew.%, 60°C): | 2,1 mPa·s |
| Leitfähigkeit: | 115 µS |
| Molekuargewicht (GPC): | 70.840 Da |

Die Fischgelatine wurde gemäß der in Beispiel 1 beschriebenen Vorgehensweise in unterschiedlichen Konzentrationen mit unterschiedlichen Mengen an Transglutaminase vernetzt. Die jeweiligen Mengenverhältnisse, Gelpunkte und Gelfestigkeiten ergeben sich aus der nachfolgenden Tabelle 6.

**Tabelle 6**

| Ansatz | 5-1 | 5-2 | 5-3 | 5-4 |
|---|---|---|---|---|
| Gelatinekonzentration | 10 Gew.% | 5 Gew.% | 15 Gew.% | 15 Gew.% |
| Menge an Gelatinelösung | 5,0 ml | 5,9 ml | 5,4 ml | 4,8 ml |
| Menge an Transglutaminase-Stammlösung | 0,3 ml | 0,5 ml | 1,0 ml | 1,6 ml |
| destilliertes Wasser | 0,9 ml | - | - | - |
| Transglutaminase pro Gramm Gelatine | 18 U/g | 51 U/g | 37 U/g | 67 U/g |
| Gelpunkt | 60 min | 30 min | 14,5 min | 9,0 min |
| Gelfestigkeit 10 min nach dem Gelpunkt | (nicht bestimmt) | 30 mN | 395 mN | 711 mN |

Auch unter Verwendung der niedrigviskosen Fischgelatine können vernetzte Gelatinegele hergestellt werden, wobei die Gelbildung im Vergleich zu hochviskosen Gelatinen erwartungsgemäß sehr langsam erfolgt und insgesamt niedrigere Gelfestigkeiten erzielt werden. Durch den Einsatz sowohl hoher Gelatinekonzentrationen als auch hoher Mengen an Vernetzungsmittel können jedoch, wie die Ansätze 5-3 und 5-4 zeigen, auch hier durchaus Werte erreicht werden, die mit den bei hochviskosen Gelatinen erreichten Werten vergleichbar sind.

Ein besonderer Vorteil bei der Verwendung von nicht gelierender Fischgelatine besteht darin, dass die Gelatinelösung bei Raumtemperatur flüssig bleibt und dadurch die Bereitsstellung und Handhabung der therapeutischen Zusammensetzung vereinfacht werden.

Durch den Einsatz von Mischungen verschiedener Gelatinetypen, z.B. von Fischgelatine mit Rinderknochen- oder Schweineschwartengelatine, ergibt sich eine weitere Möglichkeiten (neben der Variation der Gelatine- und der Vernetzungsmittelkonzentration), um den Gelpunkt und die Gelfestigkeit der vernetztenden therapeutischen Zusammensetzung zu beeinflussen.

### Beispiel 6: Vernetzung von Gelatine mit Transglutaminase: Verwendung einer partiell vernetzten Gelatine

In diesem Beispiel wurde die Gelatine zunächst einem partiellen (ersten) Vernetzungsschritt unterzogen, um die Ausgangsviskosität der Gelatinelösung zu erhöhen und um eine deutlich schnellere Gelbildung im eigentlichen (in diesem Fall zweiten) Vernetzungsschritt zu erzielen.

Als Ausgangsmaterials für die Herstellung der partiell vernetzten Gelatine diente eine Gelatine aus Schweineknochen mit einem Bloomwert von 250 g und einer Viskosität von 6,6 mPa·s (6,7 Gew.-% bei 60 °C). Eine 10 Gew.-%ige Lösung dieser Gelatine in destilliertem Wasser wurde angesetzt, indem die Gelatine zunächst 45 Minuten bei Raumtemperatur gequollen und dann für eine Stunde bei 60 °C gelöst wurde. Anschließend wurde die Lösung auf 50 °C temperiert und die entsprechende Menge der Transglutaminase-Stammlösung (30 Units/ml) zugegeben, sodass eine Menge von 1,5 Units Transglutaminase pro Gramm Gelatine vorlagen. Zur Durchführung der partiellen Vernetzung wurde die Lösung für 2 Stunden unter Rühren bei 50 °C gehalten.

Zum Abstoppen der Vernetzungsreaktion wurde die Transglutaminase durch Erwärmen der Lösung auf 80 °C thermisch deaktiviert, anschließend wurde die Lösung sofort im Eisbad abgekühlt, in eine Schale gegossen und gelieren gelassen. Das erhaltene Gelatinegel wurde gewolft, bei 20 °C und einer relativen Luftfeuchtigkeit von 10 % getrocknet und anschließend gemahlen. Die auf diese Weise erhaltene partiell vernetzte Gelatine wird im Folgenden als P2 bezeichnet.

Eine weitere partiell vernetzte Gelatine mit einem etwas höheren Vernetzungsgrad wurde wie vorstehend beschrieben hergestellt, mit der Abweichung, dass die partielle Vernetzungsreaktion für 3 Stunden durchgeführt wurde. Diese Gelatine wird im Folgenden als P3 bezeichnet.

Die Bloomwerte und Viskositäten bei 60 °C und 37 °C der Ausgangsgelatine P0 und der partiell vernetzten Gelatinen P2 und P3 sind in der nachfolgenden Tabelle 7 dargestellt.

**Tabelle 7**

| Gelatine | P0 | P2 | P3 |
|---|---|---|---|
| Bloom-Wert | 250 g | 228 g | 257 g |
| Viskosität (6,7 Gew.-%, 60 °C) | 6,6 mPa•s | 9,6 mPa•s | 16,7 mPa•s |
| Viskosität (10 Gew.-%, 37 °C) | 28,6 mPa•s | 69,8 mPa•s | 200 mPa•s |

Durch die partielle Vernetzung konnte die Viskosität der Gelatine bei 60 °C um das ca. 1,5-fache (P2) bzw. das ca. 2,5-fache (P3) gegenüber der unvernetzten Gelatine (P0) erhöht werden. Noch wesentlich deutlicher wirkt sich die Viskositätserhöhung bei 37 °C, d.h. bei einer bevorzugten Applikationstemperatur der therapeutischen Zusammensetzung, aus. Hier erhöhte sich die Viskosität um das ca. 2,5-fache bzw. das ca. 7-fache.

Unter Verwendung der Gelatinen P0, P2 bzw. P3 wurde eine Vernetzungsreaktion mit Transglutaminase durchgeführt und die Gelfestigkeit und Klebrigkeit in Abhängigkeit von der Reaktionszeit bestimmt, wie dies in Beispiel 1 beschrieben wurde. Es wurde jeweils von 5 ml einer 10 Gew-%igen Gelatinelösung ausgegangen, der jeweils 1,2 ml der Transglutaminase-Stammlösung (30 Units/ml) zugegeben wurden. Dies entspricht einer Enzymmenge von 72 Units pro Gramm Gelatine.

Die Messergebnisse sind in der nachstehenden Tabelle 8 aufgeführt.

**Tabelle 8**

| Ansatz | P0 | P2 | P3 |
|---|---|---|---|
| Gelpunkt | 1,5 min | 50 s | < 5 s |
| Gelfestigkeit nach 10 min | 1184 mN/cm² | 592 mN/cm² | 631 mN/cm² |
| Haftung nach 10 min | 316 mN/cm² | 197 mN/cm^{?} | 237 mN/cm² |

Es zeigt sich, dass durch die partielle Vernetzung der Gelatine der Gelpunkt des vernetzten Gelatinegels wesentlich schneller erreicht werden kann. Bemerkenswert ist insbesondere das mit der Gelatine P3 erzielte Ergebnis, d.h. eine fast sofortige Gelbildung innerhalb von weniger als 5 s nach dem Mischen der Gelatine mit der Transglutaminase.

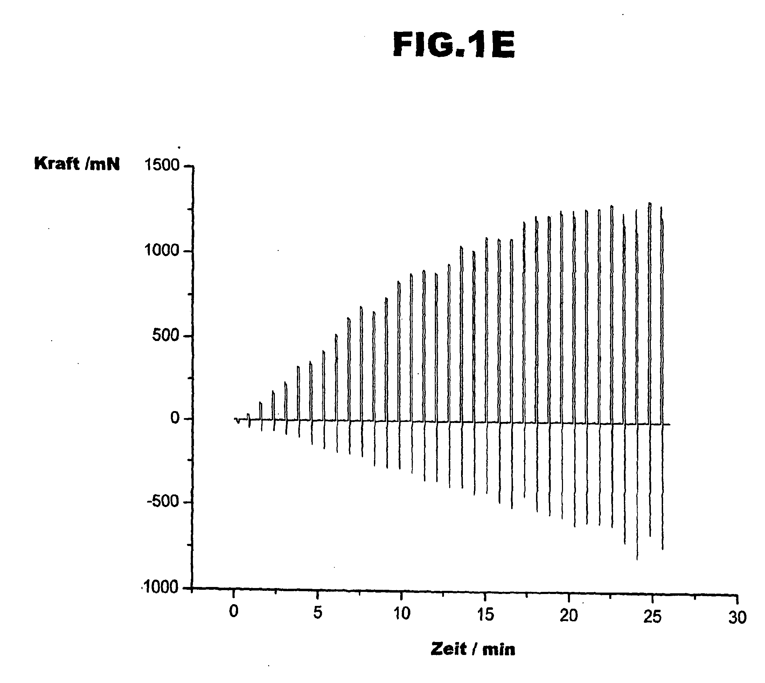

In der Figur 1E ist die gemessene Kraft in Abhängigkeit von der Reaktionszeit für den Ansatz mit der Gelatine P3 dargestellt (Durchführung der Messung wie in Beispiel 1 beschrieben). Aus der Abbildung wird deutlich, dass trotz der fast unmittelbar nach dem Mischen erfolgenden Gelierung die Gelfestigkeit und die Haftung kontinuierlich ansteigen und erst einige Zeit nach dem Gelpunkt ihren maximalen Wert erreichen. Dieser Effekt ist für die Anwendung der vorliegenden Erfindung äußerst vorteilhaft und ermöglicht es, dass die therapeutische Zusammensetzung während einem gewissen Zeitraum nach der Applikation noch plastisch verformbar ist und sich an die Struktur des Zielbereichs anpassen kann.

### Beispiel 7: Herstellung und Auflösungsverhalten einer lyophilisierten Feststoffmischung aus Gelatine und Transglutaminase

Dieses Beispiel beschreibt die Herstellung einer Feststoffmischung, die 6 Units Transglutaminase pro Gramm Gelatine enthält.

75 g der Gelatine A aus dem Beispiel 1 (Schweineschwartengelatine mit 290 g Bloom) wurden in 425 g destilliertem Wasser gequollen und bei 60°C gelöst. Die Lösung wurde auf 45°C abkühlen gelassen, mit 15 ml der Transglutaminase-Stammlösung (30 U/ml, siehe Beispiel 1) versetzt und gut durchmischt. Zwei Gefriertrocknungsschalen wurden mit flüssigem Stickstoff gekühlt, die Gelatine und Transglutaminase enthaltende Lösung darin verteilt und mittels flüssigem Stickstoff eingefroren. Die eingefrorene Lösung wurde zwei Tage in einer Gefriertrocknungsanlage vom Typ Lyovac GT 2-s (Hersteller: AMSCO Finn-Aqua GmbH, Hürth) lyophilisiert.

Die erhaltene lyophilisierte Feststoffmischung wurde unter ständiger Kühlung mit flüssigem Stickstoff in einem Mörser zu einem feinen Pulver vermahlen und anschließend unter Vakuum getrocknet. Da das Pulver stark hygroskopisch ist, wurde es unter Luftabschluss bei ca. 4°C gelagert.

Eine weitere Feststoffmischung wurde hergestellt, indem die beschriebene Vorgehensweise mit denselben Mengenverhältnissen, aber unter Verwendung einer kaltwasserlöslichen Instantgelatine an Stelle der Gelatine A wiederholt wurde. Diese Instantgelatine enthält zur Verbesserung ihrer Löslichkeit Anteile an niedermolekularem Gelatinehydrolysat.

Das Auflösungsverhalten der auf diese Weise hergestellten Feststoffmischungen wurde wie folgt untersucht: jeweils 50 mg Feststoffmischung wurden in ein verschließbares Röhrchen eingewogen und mit 950 µl PBS-Puffer (pH 7,2), der auf 37°C vorgewärmt war, versetzt. Die Röhrchen wurden mit Hilfe eines Reagenzglasschüttlers geschüttelt und die Zeit bis zur sichtbaren Auflösung der Feststoffmischung bestimmt.

Die aus der Gelatine A hergestellte Mischung war nach 2,7 min, die aus der Instantgelatine hergestellte Mischung bereits nach 2 min aufgelöst.

Das Beispiel zeigt, dass lyophilisierte Gelatine bei 37°C in einer wässrigen Lösung aufgelöst werden kann. Dies ist darauf zurückzuführen, dass die Gelatine als Ergebnis des Gefriertrocknungsprozesses überwiegend in amorpher Form vorliegt. Durch die Verwendung von Instantgelatine kann die Auflösegeschwindigkeit weiter verbessert werden.

Derartige lyophilisierte Feststoffmischungen aus Gelatine und einem Vernetzungsmittel können im Rahmen der vorliegenden Erfindung vorteilhaft eingesetzt werden. Die Mischung kann bei Raumtemperatur oder gekühlt bereitgestellt werden und dann vom behandelnden Arzt in einer wässrigen Lösung, die gegebenenfalls die zu verabreichenden Zellen enthält, bei 37°C oder weniger aufgelöst werden.

### Beispiel 8: Einbettung von porcinen Chondrozyten in ein mit Transglutaminase vernetztes Gelatinegel

In diesem Beispiel wurde die Vitalität von porcinen Chondrozyten in einem vernetzten Gelatinegel über einen Zeitraum von mehreren Tagen untersucht. Dabei wurden eine Gelatinekonzentration von ca. 17 Gew.% und eine Menge von 16 Units Transglutaminase pro Gramm Gelatine gewählt.

Eine 20 Gew.%ige Lösung der Gelatine A aus dem Beispiel 1 wurde hergestellt, indem die Gelatine bei 60°C in einer Mischung aus 35 Vol.% Hanks-Puffer und 75 Vol.% destilliertem Wasser gelöst wurde. Die Verdünnung des Hanks-Puffers mit Wasser erfolgte mit dem Ziel, die durch die Gelatine erhöhte Osmolarität der Lösung wenigstens teilweise wieder auszugleichen.

200 µl der Gelatinelösung wurden auf 37°C vorgewärmt und mit 10 µl einer Zellsuspension, die ca. 10.000 porcine Chondrozyten enthielt, sowie mit 21,3 µl Transglutaminase-Stammlösung (30 U/ml) versetzt. Die Mischung wurde auf ein Deckplättchen pipettiert und 10 min im Brutschrank bei 37°C inkubiert, wobei sich ein die Zellen enthaltendes vernetztes Gelatinegel (Zellmatrix) bildete. Dieses wurde mit 3 ml Zellkulturmedium überschichtet und weiter bei 37°C inkubiert.

Das vernetzte Gelatinegel (Zellmatrix) blieb unter diesen Bedingungen mindestens sieben Tage stabil, d.h. es bewahrte im Wesentlichen seine äußere Form. Dies zeigt, dass die therapeutische Zusammensetzung gemäß der vorliegenden Erfindung als eine stabile, dreidimensionale Matrix für die darin eingebetteten Zellen dienen kann.

Die Vitalität der Zellen wurde durch Anfärbung des vernetzten Gelatinegels mit Propidiumiodit bestimmt. Bei dieser Anfärbung werden tote Zellen im Fluoreszenzmikroskop durch eine Rotfärbung sichtbar. Bei Untersuchungen nach drei, fünf bzw. sieben Tagen zeigte sich, dass jeweils nur ein sehr geringer, im Wesentlichen konstanter Anteil an toten Zellen vorlag.

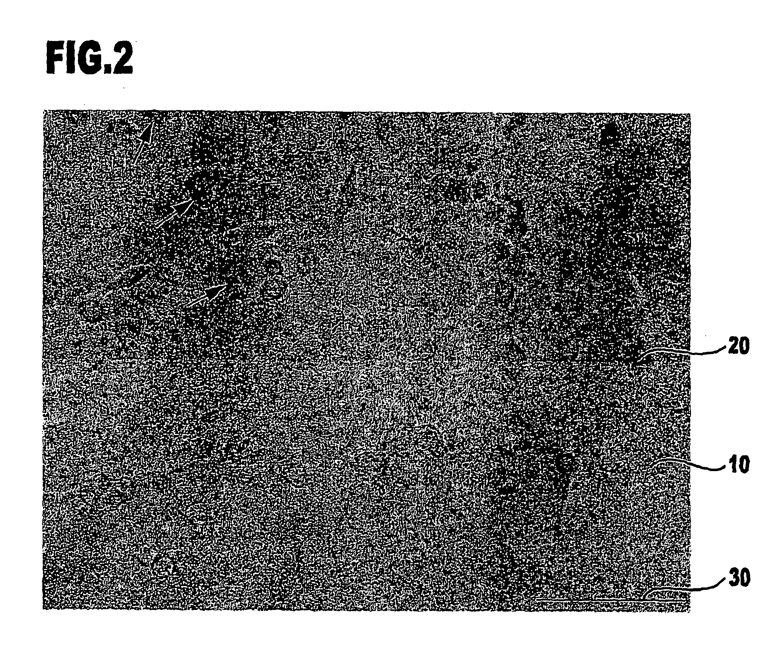

Die Figur 2 zeigt eine lichtmikroskopische Aufnahme der mit Propidiumiodit behandelten Zellmatrix eines vernetzten Gelatinegels 10 mit den darin eingebetteten Chondrozyten 20 nach einer Inkubationszeit von drei Tagen. Der Balken 30 am rechten unteren Bildrand entspricht einer Länge von 100 µm. Die toten Zellen, die im Fluoreszenzmikroskop rot erscheinen, sind durch Pfeile gekennzeichnet. In der Figur ist deutlich erkennbar, dass der überwiegende Anteil der Zellen in dem Gelatinegel nach drei Tagen noch vital ist. Ähnliche Ergebnisse wurden nach fünf bzw. sieben Tagen gefunden.

Dieses Ergebnis zeigt, dass das aus der erfindungsgemäßen Zusammensetzung gebildete Gelatinegel in der Lage ist, als eine das Zellwachstum von Säugetierzellen unterstützende Matrix zu fungieren.

### Beispiel 9: Einbettung von humanen Chondrozyten in ein mit Transglutaminase vernetztes Gelatinegel

In diesem Beispiel wurde die Wirkung des vernetzten Gelatinegels auf das Wachstumsverhalten von humanen Chondrozyten untersucht. Dabei wurden unterschiedliche Gelatinekonzentrationen bei einer gleichbleibenden Konzentration des Vernetzungsmittels Transglutaminase getestet.

Für jeden der fünf Ansätze wurden 250 µl einer Lösung der Gelatine A aus Beispiel 1 mit der jeweiligen Konzentration gemäß der nachfolgenden Tabelle 9 eingesetzt. Die Lösungen wurden in je einer Vertiefung einer 48-Well-Zellkulturplatte mit 250.000 humanen Chondrozyten beimpft, bei einer Temperatur von 40°C mit 12,5 µl Transglutaminase-Stammlösung (30 U/ml) versetzt und bei 37°C eine Woche inkubiert. Zum Vergleich wurde beim Ansatz 9-4 keine Transglutaminase zugegeben und beim Ansatz 9-5 wurde statt einer Gelatinelösung Puffer eingesetzt. Die Ergebnisse sind in der Tabelle 9 aufgeführt.

**Tabelle 9**

| Ansatz | 9-1 | 9-2 | 9-3 | 9-4 | 9-5 |
|---|---|---|---|---|---|
| Gelatinekonzentration | 12,5 Gew.% | 4,2 Gew.% | 3,0 Gew.% | 12,5 Gew.% | - |
| Transglutaminase | + | + | + | - | + |
| Transglutaminase pro Gramm Gelatine | 19 U/g | 57 U/g | 80 U/g | - | - |
| Gelpunkt | < 1 min | ca. 5 min | < 10 min | - | - |
| Ergebnis Zellbesiedlung | 3 | 2 | 1 | 2 | 4 |

Nach einer Woche Kultivierung bei 37°C wurden die Zellen mikroskopisch untersucht. Als Ergebnis der Zellbesiedlung wurden die Zelldichte und die Morphologie der Zellen beurteilt und gemäß einer Skala von 1 bis 6 bewertet, wobei 1 für eine sehr gute Zellverträglichkeit und 6 für eine Zellunverträglichkeit des vernetzten Gelatinegels steht.

Das beste Ergebnis wurde mit einer Gelatinekonzentration von 3,0 Gew.% erzielt (Ansatz 9-3). Die Zellen waren abgerundet, klein und wiesen kaum Vakuolenbildung auf. Eine Erhöhung der Konzentration auf 4,3 bzw. 12,5 Gew.% (Ansätze 9-2 und 9-1) führte tendenziell zu einer Abnahme der Zelldichte in der Matrix und zur Vergrößerung der Vakuolen.

Auch die im Vergleichsversuch (Ansatz 9-4) eingesetzte unvernetzte Gelatinelösung war für die Chondrozyten nicht erkennbar schädlich, führte jedoch zu einer ausgeprägt fibroblastenartigen Morphologie. Die Gegenwart von Transglutaminase ohne Gelatine (Ansatz 9-5) führte zu einer starken Vakuolenbildung der Zellen.

Dieser Versuch zeigt, dass durch eine geeignete Wahl der Gelatinekonzentration gute Wachstums- und Überlebensbedingungen für humane Zellen realisiert werden können.

## Patentansprüche

1. Verwendung von Gelatine und Transglutaminase zur Herstellung einer vernetzenden therapeutischen Zusammensetzung zur Behandlung von Bandscheiben- oder Meniskusschäden, wobei die Zusammensetzung ein vernetztes Gelatinegel als Zellmatrix in einem Zielbereich des menschlichen oder tierischen Körpers bildet, wobei
(i) die Gelatine und die Transglutaminase zur Bildung der vernetzenden therapeutischen Zusammensetzung miteinander gemischt werden, welche anschließend in den Zielbereich verabreicht wird; oder
(ii) die Gelatine und die Transglutaminase in voneinander getrennter Form bereitgestellt und gleichzeitig oder aufeinander folgend unter Bildung der vernetzenden therapeutischen Zusammensetzung in den Zielbereich verabreicht werden,
wobei der Zielbereich der Nucleus pulposus und/oder der Annulus fibrosus einer Bandscheibe oder ein Meniskus ist, und wobei die Zusammensetzung Bandscheibenzellen, Chondrozyten und/oder mesenchymale Stammzellen umfasst.

2. Verwendung nach Anspruch 1, wobei die Gelatine eine Fischgelatine ist.

3. Verwendung nach einem der vorangehenden Ansprüche, wobei die Zusammensetzung gemäß (i) eine wässrige Lösung ist, die die Transglutaminase und die Gelatine gelöst enthält.

4. Verwendung nach Anspruch 3, wobei die wässrige Lösung durch Auflösen einer Feststoffmischung, welche die Gelatine und die Transglutaminase in lyophillsierter Form umfasst, hergestellt wird.

5. Verwendung nach Anspruch 4, wobei die Gelatine zumindest überwiegend in amorpher Form vorliegt.

6. Verwendung nach Anspruch 4 oder 5, wobei die Transglutaminase in einer Menge von 0,6 bis 80 Units pro Gramm Gelatine in der Feststoffmischung enthalten ist.

7. Verwendung nach Anspruch 6, wobei die Transglutaminase in einer Menge von 5 bis 40 Units pro Gramm Gelatine in der Feststoffmischung enthalten ist.

8. Verwendung nach Anspruch 1 oder 2, wobei das Bereitstellen gemäß (ii) in Form einer wässrigen Gelatinelösung und einer separaten wässrigen Transglutaminaselösung erfolgt.

9. Verwendung nach Anspruch 8, wobei die gleichzeitige Verabreichung der Gelatinelösung und der Transglutaminaselösung durch Injektion mit einer Mehrkammerapplikationsvorrichtung erfolgt.

10. Verwendung nach Anspruch 1 oder 2, wobei das Bereitstellen gemäß (ii) in Form einer wässrigen Gelatinelösung und der Transglutaminase in fester Form erfolgt.

11. Verwendung nach einem der vorangehenden Ansprüche, wobei die Gelatinekonzentration in der Zusammensetzung 5 bis 20 Gew.% beträgt.

12. Verwendung nach einem der vorangehenden Ansprüche, wobei das vernetzte Gelatinegel, das die Zellmatrix bildet, eine Gelfestigkeit von 100 g oder mehr aufweist, gemessen mit einem Stempel mit einem Durchmesser von 12,7 mm bei einer Eindringtiefe von 4 mm.

13. Verwendung nach einem der vorangehenden Ansprüche, wobei die Gelatine vor dem Mischen und/oder Verabreichen gemäß (i) oder (ii) partiell vernetzt ist.

14. Verwendung nach Anspruch 13, wobei die Gelatine unter Verwendung von Transglutaminase partiell vernetzt ist.

15. Verwendung nach einem der vorangehenden Ansprüche, wobei die Zusammensetzung Wachstumsfaktoren, Differenzierungsfaktoren, entzündungshemmende Substanzen und/oder antibiotisch wirkende Substanzen umfasst.

16. Feststoffmischung, welche Gelatine und Transglutaminase in lyophilisierter Form umfasst.

## Claims

1. Use of gelatin and transglutaminase for producing a cross-linking therapeutic composition for treating intervertebral disc damage or meniscus damage, the composition forming a cross-linked gelatin gel as a cell matrix in a target area of the human or animal body, where
(i) the gelatin and the transglutaminase are mixed with one another to form the cross-linking therapeutic composition, which is subsequently administered to the target area; or
(ii) the gelatin and the transglutaminase are provided in mutually separate form and are administered simultaneously or consecutively to the target area with formation of the cross-linking therapeutic composition,
the target area being the nucleus pulposus and/or the annulus fibrosus of an intervertebral disc, or a meniscus, and the composition comprising intervertebral disc cells, chondrocytes and/or mesenchymal stem cells.

2. Use according to Claim 1, where the gelatin is a fish gelatin.

3. Use according to either of the preceding claims, where the composition according to (i) is an aqueous solution which comprises the transglutaminase and the gelatin in solution.

4. Use according to Claim 3, where the aqueous solution is produced by dissolving a solids mixture which comprises the gelatin and the transglutaminase in lyophilized form.

5. Use according to Claim 4, where the gelatin is present at least predominantly in amorphous form.

6. Use according to Claim 4 or 5, where the transglutaminase is contained in the solids mixture in a quantity of 0.6 to 80 units per gram of gelatin.

7. Use according to Claim 6, where the transglutaminase is contained in the solids mixture in a quantity of 5 to 40 units per gram of gelatin.

8. Use according to Claim 1 or 2, where the providing according to (ii) takes place in the form of an aqueous gelatin solution and a separate aqueous transglutaminase solution.

9. Use according to Claim 8, where the simultaneous administration of the gelatin solution and of the transglutaminase solution takes place by injection using a multi-chamber applicator.

10. Use according to Claim 1 or 2, where the providing according to (ii) takes place in the form of an aqueous gelatin solution and of the transglutaminase in solid form.

11. Use according to any of the preceding claims, where the gelatin concentration in the composition amounts to 5 to 20% by weight.

12. Use according to any of the preceding claims, where the cross-linked gelatin gel that forms the cell matrix has a gel strength of 100 g or more, measured with a plunger having a diameter of 12.7 mm at a penetration depth of 4 mm.

13. Use according to any of the preceding claims, where the gelatin is partially cross-linked before the mixing and/or administration according to (i) or (ii).

14. Use according to Claim 13, where the gelatin is partially cross-linked using transglutaminase.

15. Use according to any of the preceding claims, where the composition comprises growth factors, differentiation factors, anti-inflammatory substances and/or antibiotic substances.

16. Solid mixture which comprises gelatin and transglutaminase in lyophilized form.

## Revendications

1. Utilisation de gélatine et de transglutaminase pour la préparation d'une composition thérapeutique réticulante pour le traitement de lésions aux disques intervertébraux ou aux ménisques, la composition formant un gel de gélatine réticulé comme matrice cellulaire dans une zone cible du corps humain ou animal, où
(i) la gélatine et la transglutaminase sont mélangées les unes avec les autres pour la formation de la composition thérapeutique réticulante, qui est ensuite administrée dans la zone cible ; ou
(ii) la gélatine et la transglutaminase sont préparées sous une forme séparée l'une de l'autre et administrées simultanément ou consécutivement dans la zone cible avec formation de la composition thérapeutique réticulante,
où la zone cible est le nucleus pulposus et/ou l'annulus fibrosus (anneau fibreux) d'un disque intervertébral ou un ménisque et où la composition comprend des cellules de disque intervertébral, des chondrocytes et/ou des cellules souches du mésenchyme.

2. Utilisation selon la revendication 1, où la gélatine est une gélatine de poisson.

3. Utilisation selon l'une quelconque des revendications précédentes, où la composition selon (i) est une solution aqueuse, qui contient la transglutaminase et la gélatine sous forme dissoute.

4. Utilisation selon la revendication 3, où la solution aqueuse est préparée par dissolution d'un mélange de solides, qui comprend la gélatine et la transglutaminase sous forme lyophilisée.

5. Utilisation selon la revendication 4, où la gélatine se trouve au moins principalement sous forme amorphe.

6. Utilisation selon la revendication 4 ou 5, où la transglutaminase est contenue en une quantité de 0,6 à 80 unités par gramme de gélatine dans le mélange de solides.

7. Utilisation selon la revendication 6, où la transglutaminase est contenue en une quantité de 5 à 40 unités par gramme de gélatine dans le mélange de solides.

8. Utilisation selon la revendication 1 ou 2, où la préparation selon (ii) a lieu sous forme d'une solution aqueuse de gélatine et d'une solution aqueuse séparée de transglutaminase.

9. Utilisation selon la revendication 8, où l'administration simultanée de la solution de gélatine et de transglutaminase a lieu par injection avec un dispositif d'administration à plusieurs chambres.

10. Utilisation selon la revendication 1 ou 2, où la préparation selon (ii) a lieu sous forme d'une solution aqueuse de gélatine et de la transglutaminase sous forme solide.

11. Utilisation selon l'une quelconque des revendications précédentes, où la concentration en gélatine dans la composition est de 5 à 20% en poids.

12. Utilisation selon l'une quelconque des revendications précédentes, où le gel réticulé de gélatine, qui forme la matrice cellulaire, présente une résistance du gel de 100 g ou plus, mesurée avec un piston présentant un diamètre de 12,7 mm à une profondeur de pénétration de 4 mm.

13. Utilisation selon l'une quelconque des revendications précédentes, où la gélatine est réticulée partiellement avant le mélange et/ou l'administration selon (i) ou (ii).

14. Utilisation selon la revendication 13, où la gélatine est réticulée partiellement avec utilisation de transglutaminase.

15. Utilisation selon l'une quelconque des revendications précédentes, où la composition comprend des facteurs de croissance, des facteurs de différenciation, des substances anti-inflammatoires et/ou des substances à effet antibiotique.

16. Mélange solide, qui comprend de la gélatine et de la transglutaminase sous forme lyophilisée.
